(19) Europäisches Patentamt
European Patent Office
Office européen des brevets

(11) EP 2 850 208 B1

(12) EUROPEAN PATENT SPECIFICATION

(45) Date of publication and mention
of the grant of the patent:
**19.07.2017 Bulletin 2017/29**

(51) Int Cl.:
*C12Q 1/68* (2006.01)     *G01N 33/50* (2006.01)

(21) Application number: **13731426.6**

(22) Date of filing: **17.05.2013**

(86) International application number:
**PCT/GB2013/051285**

(87) International publication number:
**WO 2013/171508 (21.11.2013 Gazette 2013/47)**

(54) **METHOD FOR DETERMINING IF A SUBJECT HAS AN INCREASED LIKELIHOOD OF HIGH GRADE DYSPLASIA OR ESOPHAGAL ADENOCARCINOMA**

VERFAHREN ZUR FESTSTELLUNG DER ERHÖHTEN WAHRSCHEINLICHKEIT HOCHGRADIGER DYSPLASIE ODER EINES SPEISERÖHRENADENOKARZINOMS BEI EINER PERSON

PROCÉDÉ POUR DÉTERMINER SI UN SUJET PRÉSENTE UNE PROBABILITÉ AUGMENTÉE DE DYSPLASIE DE GRADE ÉLEVÉ OU D'ADÉNOCARCINOME OESOPHAGIEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.05.2012 GB 201208963**

(43) Date of publication of application:
**25.03.2015 Bulletin 2015/13**

(73) Proprietors:
• **Medical Research Council**
  **Swindon SN2 1FL (GB)**
• **Cambridge Enterprise Limited**
  **Cambridge CB2 1TN (GB)**

(72) Inventors:
• **DI PIETRO, Massimiliano**
  **Cambridge**
  **Cambridgeshire CB2 2XZ (GB)**
• **FITZGERALD, Rebecca**
  **Cambridge**
  **Cambridgeshire CB2 2XZ (GB)**
• **LIU, Xinxue**
  **Cambridge**
  **Cambridgeshire CB2 2XZ (GB)**

(74) Representative: **Script IP Limited**
  **Turnpike House**
  **18 Bridge Street**
  **Frome Somerset BA11 1BB (GB)**

(56) References cited:
**WO-A1-2013/041684      WO-A2-2011/133935**

• **KAYE PHILIP V ET AL: "Barrett's dysplasia and the Vienna classification: reproducibility, prediction of progression and impact of consensus reporting and p53 immunohistochemistry", HISTOPATHOLOGY (OXFORD), vol. 54, no. 6, May 2009 (2009-05), pages 699-712, XP009173235, ISSN: 0309-0167**
• **P C GALIPEAU ET AL: "clonal expansion and loss of heterozygosity at chromosome 9p and 17p in premalignant esophageal (Barrett's) tissue", J. NAT. CANCER INST., vol. 91, no. 24, 15 December 1999 (1999-12-15), pages 2087-2095, XP55082745,**
• **KARSTEN SCHULMANN ET AL: "Inactivation of p16, RUNX3, and HPP1 occurs early in Barrett's-associated neoplastic progression and predicts progression risk", ONCOGENE, vol. 24, no. 25, 11 April 2005 (2005-04-11), pages 4138-4148, XP55082746, ISSN: 0950-9232, DOI: 10.1038/sj.onc.1208598**
• **P. LAO-SIRIEIX ET AL: "Cyclin A Immunocytology as a Risk Stratification Tool for Barrett's Esophagus Surveillance", CLINICAL CANCER RESEARCH, vol. 13, no. 2, 15 January 2007 (2007-01-15), pages 659-665, XP55082747, ISSN: 1078-0432, DOI: 10.1158/1078-0432.CCR-06-1385**

**Description**

**Field of the Invention**

The invention relates to the detection or prediction of high grade dysplasia (HGD) and/or esophageal adenocarcinoma (EC).

**Background to the invention**

Esophageal Adenocarcinoma (EA or EC) has seen a dramatic 6-fold rise in incidence rate over the last 30 years [1]. Despite technological advances in diagnosis and refinement of multi-disciplinary treatment approaches, survival rates remain dismal with about 12% patients alive at 5 years after the diagnosis of EA[2]. The only recognized precursor to EA is Barrett's Esophagus (BE), which is believed to progress to cancer through intermediate dysplastic stages [3, 4]. Improvement in survival rates clearly ground on the ability to diagnose early, therefore all major professional societies recommend endoscopic surveillance with four-quadrant random biopsies every 2 cm (Seattle protocol) plus targeted biopsies on any visible lesion (BSG guidelines) [4-8].

Diagnosis of dysplasia or early cancer allows endoscopic treatment of BE with preventive strategies for the development of advanced disease (Wiesbaden on EMR) [9, 10], However, endoscopic surveillance has major limitations. The Seattle protocol is time consuming due to the number of biopsies involved. This generates significant costs to the health care and makes the adherence to the endoscopic guidelines poor in the endoscopic community. In addition dysplasia and early EA can be inconspicuous at endoscopy.

The current standard for endoscopic sampling (surveillance for Barrett's oesophagus) is to follow the Seattle Protocol. This well established technique requires quadruple sampling at sites evenly spaced around the oesophagus. This sampling is repeated every two centimetres for the entire length of the oesophagus. This is not a targeted sampling - the samples are taken at the prescribed distances independent of any observations which might be made. This technique has several disadvantages. This is very time consuming, and involves a large number of samples being collected. There is a risk of bleeding, which risk is amplified due to the large number of samples being collected. There is poor adherence to the protocol, which can cause problems in analysis. This procedure requires a lot of time for a trained endoscopist and there is a significant cost burden for the health care provider. This technique also involves sampling errors which can be difficult to resolve. In addition, there is poor agreement amongst histopathologists on the classification and assessment of dysplasia.

The use of auto fluorescence imaging (AFI) within endoscopic tri-modal imaging (ETMI) can improve dysplasia detection. However, this technique has drawbacks too. Firstly, this technique has a high false positive rate. Indeed, the false positive rate with AFI can be as high as 80%. It has been established that ETMI cannot replace random biopsies. Therefore, this technique has the limitation of being no better than the prior art random sampling approach.

Biomarkers have been proposed to improve risk stratification in Barrett's oesophagus (BO or BE), however the molecular heterogeneity of BE can hamper the detection of molecular changes in random biopsies. The use of Autofluorescence Imaging (AFI) within endoscopic Trimodal Imaging (ETMI) can improve dysplasia detection, but has a high false positive rate.

Blind quadrantic biopsies are recommended in absence of visible lesions, but these only represent approximately 5 % of the BE surface, and therefore sampling error is inevitable [11]. Even when dysplasia is found, its diagnosis and grading can be subjective with a high inter-observer variability among both general pathologist and expert pathologists [12] [13] [14]. For all these reason the cost-effectiveness of endoscopic surveillance for BE has been questioned, especially following recent population studies and a meta-analysis which have suggested that the cancer risk in BE may be lower than previously, lying somewhere between 0.12 and 0.38% per year. To overcome the limitations related to the poor endoscopic detection of dysplasia a number of enhanced endoscopic modalities have been investigated. High resolution endoscopy (HRE)has dramatically improved the image quality [19], however to date there is no definite evidence that this can improve dysplasia detection in BE. Magnification endoscopy in conjunction with chromoendoscopy or virtual chromoendoscopy, such narrow band imaging (NBI), has certainly improved the inspection of the mucosal pit and vessel pattern towards predicting the histological outcome [19], however definite evidence that these techniques can increment the diagnostic yield on a per patient basis is lacking (cite me-analysis for chromoendoscopy and Sharma paper for NBI). In the attempt to find a good red-flagging technique for dysplasia, auto-fluorescence imaging (AFI) has been studied. AFI relies on the fact that some molecules contained in the supeficial layers of the GI tract called flurophores can emit green fluorescence when excited by short wave-lengths, such as blue light. Dysplasia and early cancer can be associated to loss of green autofluorescence due to both distortion of tissue architecture, such as cellular crowding and increased vasculature, and molecular abnormalities, such as reduced collagen and increased DNA content, which occur in neoplasia [22]. In a uncontrolled feasibility study AFI detected 11 additional HGD/AC lesions and 7 additional patients with HGD/AC when compared to WLE, at the price of a 50% false-positive rate [23]. To improve this low specificity, AFI has been incorporated with HRE and NBI into a single endoscope called Endoscopic Trimodal Imaging (ETMI) ETMI has been tested in randomized controlled studies both in tertiary and primary care. These showed that although ETMI can increase detection rate of dysplasia, mainly due to the AFI mode, AFI had a false positive rate of about 80% and the overall diagnostic yield of dysplasia compared to standard endoscopy with the Seattle protocol was not significantly different [24, 25]. Although inflammation has been proposed to justify the AFI false-positivity, it remains unclear whether this might rely at least in part to molecular changes which intervene in the BE carcinogenetic process.

In parallel to the above mentioned technological advancement, researchers have been investigating over the last 10 years the use of molecular abnormalities to improve risk stratification in BE and to make this more objective that the pathological assessment of dysplasia. The identification of patients at higher risk to progress to cancer through the use of molecular biomarkers has the clear advantage to allow focusing the surveillance strategies on high-risk individuals with less intensive monitoring of a larger proportion of low-risk individuals and ultimate benefit to the health care finances. So far a number of different biomarkers have been investigated in phase 3 studies. Overexpression p53 on index biopsies was strongly predictive of risk of progression with a hazard ratio (HR) = 3.8 (95% CI, 1.5-10) [26], which has been confirmed by other studies [27-29]. Surface expression cyclin A has also been showed to be predictive of future with a relative risk (RR) of 7.6 (95%CI, 1.6-37.0). Schulmann and coworkers have found that methylation at p16, RUNX3 and HPP1 genes correlated with risk of progression with odds ratio (OR) 1.74 (95% CI, 1.33-2.20), 1.80 (95% CI, 1.08-2.81) and 1.77 (95% CI, 1.06-2.81) respectively [30]. These three genes when combined in a model could predict progression risk to HGD/AC 2 years before their development and in another study by same group the model could predict 50% of patients who progressed to HGD/EA. Finally, Galipeau et al found that 17p and 9p LOH, tetraploidy and aneuploidy individually associated with increased risk of progression with RR = 10.6 (95% CI, 5.2-21.3), RR = 8.8 (95% CI, 4.3-17.7), RR = 8.5 (95% CI, 4.3-17.0), RR = 2.6 (95% CI, 1.1.-6.0) respectively and much higher RR when combined in a panel [31]. Despite promising, these biomarkers have only been tested by single groups in retrospective cohorts of patients and this has hampered their translation into clinical practice. In addition, these biomarkers have been normally tested on single or few random biopsies taken within the BE. Since areas of molecular abnormality in BE can be patchy and heterogeneous, this could be subjected by the same sampling bias as per dysplasia.

D1 (Kaye PV, Haider SA, Ilyas M, et al. Histopathology 2009;54:699-712) discloses Barrett's dysplasia and the Vienna classification: reproducibility, prediction of progression and impact of consensus reporting and p 53 immuno histo c he mistry.

D2 (Galipeau PC, Prevo LJ, Sanchez CA, et aL J Natl Cancer Inst 1999;91:2087-95) discloses Clonal expansion and loss of heterozygosity at chromosomes 9p and 17p in p re m a lig na nt e so p ha g e al (Barrett's) tissue.

D3 (Schulmann, K, et al., Oncogene, 2005. 24(25): p. 4138-48) discloses Inactivation of p16, RUNX3, and HPP1 occurs early in Barrett's-associated neoplastic progression and predicts progression risk.

D4 (Lao-Sirieix, P., L Lovat, and R.C. Fitzgerald, Clin Cancer Res, 2007. 13(2 Pt 1): p. 659-65) discloses Cyclin A immuno c yto lo gy a s a risk stratific a tio n tool for Barrett's e so p ha g us surveillance.

D6 (WO2011/133935) discloses methods and kits for risk assessment of Barrett's neoplastic progression.

The present invention seeks to overcome problem(s) associated with the priorart.

## Summary of the Invention

In the prior art, auto fluorescence Imaging (AFI) has been used to direct biopsies. This technique has been used to direct the location of biopsies to be used for grading of lesions by histopathology. In the prior art, it had been hoped that AFI patches would correlate with dysplasia, but this has not been the case. For example, in the priorart, dysplastic areas may be observed both inside and outside of AFI patches. In the prior art, AFI targeted biopsie s have been shown to be no better than random biopsies.

By contrast, the present inventors have been able to show that AFI targeted biopsies can be very useful when combined with a particular panel of biomarkers disclosed herein. The inventors have studied a range of biomarkers and have settled upon an empinical group which form a panel. The inventors find that detecting the presence of two or more biomarkers from the defined panel described herein can be indicative of high grade dysplasia (HGD) or esophagaladenoccarcinoma (EC).

More specifically, the inventors teach that there is a special advantage to analysing the biomarkers in the disclosed panel on samples which have been taken from AFI patches of the subject of interest.

It has been a problem in the art that screening for AFI patches only leads to false positives and does not represent a reliable method of diagnosing high grade dysplasia (HGD) and/or esophagaladenocarcinoma (EC). The inventors have surprisingly shown that by combining a specific panel of biomarkers with sample taken from AFI patches, a dramatic reduction in the number of false positives indicated by AFI alone can be achieved. Moreover, the invention brings further significant benefits such as a dramatically decreased number of samples (e.g. biopsies) needed to be taken to achieve the same reliable result.

Thus, in one aspect the inve ntio n provides a method for determining if a subject has an increased likelihood of high grade dysplasia (HGD) or esophagal adenocarcinoma (EC), the method comprising;

a) providing a sample from an AFI positive patch of said subject's oesophagus
b) assaying said sample for

(i) aneuploidy, and
(ii) abnormal p53, wherein assaying for p53 comprises immuno histo chemical staining of the sample fo r p 53, and scoring the stain as normal or abnormal wherein either a strong, dark stain compared to background stain or an absence of stain compared to background stain is an abnormal stain; and
(iii) abnormal Cyclin A, wherein Cyclin A is assayed by immuno histochemical staining of the sample forCyclin A, determining the percentage of Cyclin A positive epithelial surface cells compared to Cyclin A negative epithelial surface cells, wherein a percentage of 1% or more Cyclin A positive epithelial surface cells is an abnormal stain;

c) wherein if said sample shows the presence of at least two of(i),(ii)and (iii),then said subject is determined as having an increased like lihood of high grade dysplasia (HGD) or esophagaladenocarcinoma (EC).

Suitably said sample is further assayed for one or more of 9p LOH, Runx3, p16, 17p LOH, HPP1 or G2.

Suitably assay of Runx3, p16, HPP1 means determination of methylation of said gene(s); suitably determination of hypermethylation of said genes.

Suitably assay of G2 means assessment of ploidy, suitably determination of tetraploidy.

Suitably said sample is a biopsy.

Suitably said sample is an endoscopic sample.

Suitably aneuploidy is assayed by determining the DNA content by flow cytometry DNA analysis and inferring from said detemination whether said DNA content is aneuploid.

Suitably p53 is assayed by immunohisto chemistry.

Suitably Cyclin A is assayed by determining the percentage of surface cells staining positive for Cyclin A.

Also described is a method of treatment of a subject comprising determining if a subject has an increased like lihood of high grade dysplasia (HGD) or esophagal adenocarcinoma (EC) as described above, wherein if said subject is determined as having an increased like lihood of high grade dysplasia (HGD) or esophagal adenocarcinoma (EC) then endoscopic mucosal resection (EMR) and/or radio frequency a b la tio n (RFA) a re applied to sa id subject. Suita b ly ra d io frequency a b la tio n (RFA) is applied to sa id subject.

Suitably the invention is carried out using multivariate analysis. In other words, the presence of two or more of the core panel of three markers is required. The results depend on the simultaneous presence of those at least two markers; presence of me re ly one m a rke r (univa ria te a na lysis) is less reliable.

## Detailed Description of the Invention

We describe TIME (Trimodal Imaging and Molecular Endpoints) - A multi-centre prospective study combining Endoscopic Trimodal imaging and Molecular Endpoints to improve risk stratification in Barrett's Esophagus.

## Definitions

The term 'comprises' (comprise, comprising) should be understood to have its normal meaning in the art, i.e. that the stated feature or group of features is included, but that the term does not exclude any other stated feature or group of features from also being present.

Abbreviations: AIC, Akaike information criterion; AFI, Autofluorescence Imaging; AUC, area under the ROC curve; BE, Barrett's esophagus; DMSO, dimethylsulfoxide; EA, esophageal adenocarcinoma; EC, early cancer; FACS, fluorescence activated cell sorting; GI, gastro-intestinal; HGD, high grade dysplasia; IC, image cytometry; ID, indefinite for dysplasia; IHC, immunohistochemistry; LGD, low grade dysplasia; LOH, loss of heterozigosity; MI, multiple imputation; N/A, not applicable; NDBE, non-dysplastic Barrett's esophagus; ROC, receiver operating characteristic.

Abnormal vs Normal

It is believed that the skilled reader can determine whether or not the markers are normal or abnormal using the guidance given herein. However, in case further explanation is helpful, further details may be taken from the examples section and the data presented in support of the examples.

The term 'mutant' of a biomarker such as a polypeptide biomarker of the invention should have its normal meaning in the art. Mutants are sometimes referred to as 'variants' or 'alleles'. The key is to detect biomarkers as have been set out herein. The biomarkers may possess individual variations in the form of mutations or allelic variants between individuals being studied. Therefore there may be some degree of deviation from the exemplary SEQ ID NOs provided herein. The SEQ ID NOs provided herein are to assist the skilled reader in identifying and working with the polypeptides/biomarkers of the invention and are not intended as a restricted and inflexible definition of the individual polypeptides being assayed. Thus minor sequence differences between the SEQ ID NOs provided and the actual sequences of the polypeptide biomarkers being detected will be expected within the boundaries of normal variation between subjects. This should not affect the working of the invention.

Fragments/Peptides

It will be appreciated by the skilled worker that the details of the biomarkers discussed herein and in particular the sequences presented for them are given to facilitate their detection. The important information being gathered is the presence or absence (or particular level) of the biomarker in the sample being studied. There is no particular requirement that the full length polypeptide be scored. Therefore the invention embraces the detection of fragments of the polypeptide/lectin biomarkers. Moreover, the kits and peptides of the invention may comprise fragments of the polypeptides and need not comprise the full length sequences exemplified herein.

Thus a fragment is suitably at least 6 amino acids in length, suitably at least 7 amino acids in length, suitably at least 8 amino acids in length, suitably at least 9 amino acids in length, suitably at least 10 amino acids in length, suitably at least 15 amino acids, suitably at least 25 amino acids, suitably at least 50 amino acids, suitably at least 100 amino acids, or suitably the majority of the biomarker polypeptide of interest.

**Sample**

The sample may be from a subject. The subject is suitably a mammal, most suitably a human.

In one embodiment the method of the invention may comprise the step of collecting the sample. Suitably the sample is collected from an AFI patch of the subject's oesophagus. Suitably the sample is collected using an endoscope. Suitably the sample is a conventional 'pinch' sample or biopsy taken during endoscopy.

Suitably the methods do not involve actual collection of the sample. Suitably the sample is an *in vitro* sample.

Suitably the sample is an extracorporeal sample.

Methods of the invention are suitably performed on an isolated sample from the subject being investigated. Thus, suitably the methods are methods which may be conducted in a laboratory setting without the need for the subject to be present. Suitably the methods are carried out *in vitro* i.e. suitably the methods are *in vitro* methods.

In one embodiment suitably the method is an *in vitro* method. In one embodiment suitably the method is an extracorporeal method. In one embodiment suitably the actual sampling of the cells is not part of the method of the invention. Suitably the method does not involve collection of the cells. Suitably the sample is a sample previously collected. Suitably the method does not require the presence of the subject whose cells are being assayed. Suitably the sample is an *in vitro* sample.

Suitably the method of the invention is conducted *in vitro*. Suitably the method of the invention is conducted extracorporeally.

Suitably the sample is from a subject having Barrett's Esophagus.

Suitably the sample comprises material taken from an auto florescent imaging (AFI) positive region of the subject's oesophagus. This may be from a region known to show autofluorescence. This may be from a region which exhibits autofluorescence during endoscopy.

A region of the oesophagus which is observed to show autofluorescence is termed an 'AFI patch' or 'AFI positive' or 'AFI positive patch'. Suitably the sample is from an AFI patch.

The AFI positive sample may comprise material taken from the Barrett's Esophagus segment itself.

The AFI positive sample may comprise dysplastic cells.

Suitably the sample is a biopsy.

Suitably the sample comprises formalin fixed paraffin embedded (FFPE) material. It is an advantage of the invention that the methods can be carried out on FFPE material. Clinically obtained samples are often processed into FFPE material. Therefore the invention may be practiced very widely.

Suitably each of the markers described herein may be detected in FFPE material. Although there are a range of techniques for assaying the markers described, most suitably those techniques which can be carried out on FFPE material are used. This provides the advantage that only one sample is needed in order to assess multiple markers as described herein.

**Reference Standard**

The reference standard typically refers to a sample from a healthy individual i.e. one who does not have HGD and/or EC. The reference standard may be from a healthy individual who has BE but does not have HGD/EC, most suitably does not have EC. Moreover, controls may be chosen with greater precision depending on which marker is being considered. For example if aneuploidy is being considered then it may be advantageous to choose a control of any normal tissue (normal squamous oesophagus for example).

The reference standard can an actual sample analysed in parallel. Alternatively the reference standard can be one or more values previously derived from a comparative sample e.g. a sample from a healthy subject. In such embodiments a mere numeric comparison may be made by comparing the value determined for the sample from the subject to the numeric value of a previously analysed reference sample. The advantage of this is not having to duplicate the analysis by determining concentrations in individual reference samples in parallel each time a sample from a subject is analysed.

Suitably the reference standard is matched to the subject being analysed e.g. by gender e.g. by age e.g. by ethnic background or other such criteria which are well known in the art. The reference standard may be a number such as an absolute concentration drawn up by one or more previous studies.

Reference standards may suitably be matched to specific patient sub-groups e.g. elderly subjects, or those with a previous relevant history such as acid reflux or BE.

Suitably the reference standard is matched to the sample type being analysed. For example the concentration of the biomarker polypeptide(s) being assayed may vary depending on the type or nature of the sample. It will be immediately apparent to the skilled worker that the concentration value(s) for the reference standard should be for the same or a comparable sample to that being tested in the method(s) of the invention. For example, if the sample being assayed is from the Barrett's segment then the reference standard value should be for Barrett's segment to ensure that it is capable of meaningful cross-comparison. Suitably the sample type for the reference standard and the sample type for the subject of interest are the same.

Where (if) H scores are determined for staining of the sample, determination of whether the stain is normal or abnormal is suitably carried out by comparing the determined H score to a reference standard H score (such as the most frequent median distribution amongst sample(s) from control subjects) and inferring from said comparison whether said staining is abnormal.

**Advantages**

In one aspect, the invention relates to a method of determining a surveillance interval for a subject suspected of having a Barrett's associated lesion such as high grade dysplasia (HGD) and/or esophagal adenocarcinoma (EC), the method comprising assaying the subject for the presence of auto florescent imaging (AFS) patches within their oesophagus, wherein if no AFI patches are observed then the surveillance interval selected is two to five years, suitably five years; wherein if AFI patches are detected then the surveillance interval selected is less than two years.

In another aspect, the invention relates to a method for selecting the areas of an oesophagus to sample from a subject, the method comprising assaying the subject's oesophagus for AFI patches, wherein biopsies are taken only from the location of said AFI patches.

In some aspects, the invention may be regarded as a secondary tool for stratification or risk analysis of a patient already suspected of having Barrett's oesophagus.

Suitably the biomarker analysis described herein is practiced on the samples which are targeted to specific areas of the oesophagus such as AFI patches. Oesophagal sampling techniques which non-specifically sample the whole surface of the oesophagus such as the cytosponge approach, may not be suitable for use in this invention since those approaches do not preserve position or information in the cells collected, or are not easily targeted for example to AFI patches for sample collection.

In the prior art, AFI has been dismissed for producing to many false positives. However, according to the invention, even using the method described herein on a patient presenting with a false positive result for AFI saves labour and saves invasive techniques being deployed on that subject. This is because according to the invention samples are suitably targeted from AFI patches. Compared to the standard approach of quadruple sampling every two centimetres of the oesophagus, instead the inventors teach that merely sampling the AFI patches provides a robust and reliable result.

**Panel**

Suitably the panel of biomarkers comprises at least three biomarkers, said at least three biomarkers being aneuploidy, p53 and Cyclin A.

Optionally, the method of the invention may be applied using more than three biomarkers. Each time the method is applied, suitably at least aneuploidy, p53 and Cyclin A are assayed; suitably the fourth and any further biomarkers in embodiments employing more than three biomarkers are in addition to aneuploidy, p53 and Cyclin A.

The markers discussed are presented below in descending order of preference, 4 being the highest and 9 being the lowest. In a strict sense, the markers are ranked to predict any dysplasia. When the markers are tied on rank (i.e. for those markers having the same rank) then suitably the markers are preferred in descending order as presented below:

| core panel | p53 staining |
| --- | --- |
| core panel | CyclinA |
| core panel | Aneuploidy |
| 4 | 9p LOH |
| 5 | Runx3 |
| 5 | p16 |
| 5 | 17p LOH |
| 8 | HPP1 |
| 9 | G2 |

The advantage of assaying of extra markers in addition to the core panel of three is the provision of extra sensitivity with extra markers.

For markers 4 to 9, they are suitably added to the core panel of 3 markers in descending order relative to their presentation above (i.e. ascending order of rank from lowest to highest from 4 to 9).

Thus suitably the methods of the invention comprise further assaying at least one further marker from 4 to 9 above; suitably at least 2 further markers from 4 to 9 above, suitably at least 3 further markers from 4 to 9 above, suitably at least 4 further markers from 4 to 9 above, suitably at least 5 further markers from 4 to 9 above, suitably all 6 further markers from 4 to 9 above.

In some embodiments, most suitably when further markers are added as above, they are added in a 'ratchet' or 'strict additive' manner i.e. suitably if adding one further marker, this would be 4 (9p LOH); if adding two further markers these would be 4 (9p LOH) and 5 (Runx3); if adding four further markers these would be 4 (9p LOH) and 5 (Runx3) and 5 (p16) and 5 (17p LOH) and so on.

Alternatively, the further marker(s) may be added in an order or grouping of the operator's choice.

In order to provide a suitable level of confidence (i.e. statistical significance) at least two of the preferred panel of three biomarkers of aneuploidy, p53 and Cyclin A must be found to be positive in a sample to indicate an increased likelihood of presence of high grade dysplasia (HGD) or esophagal adenocarcinoma (EC).

It should be noted that the 3-biomaker panel (score ranging from 0-3) has a more accurate predictive ability to detect HGD/EC than any dysplasia. In addition, subjects with 2 or 3 biomarkers abnormal are also at higher risk of having low grade dysplasia (LGD) compared to those with 0 or 1 abnormal biomarker. Thus in some aspects the invention may relate to a method for detecting LGD in a subject, the method comprising

a) providing a sample from an AFI patch of said subject's oesophagus
b) assaying said sample for

(i) aneuploidy, and
(ii) abnormal p53, and
(iii) abnormal Cyclin A

c) wherein if said sample shows the presence of at least two of (i), (ii) and (iii), then said subject is determined as having an increased likelihood of low grade dysplasia (LGD).

The invention may also be applied to risk stratification. For example, the risk of having any dysplasia and/or HGD and/or EC is increasing with the increase of number of abnormal biomarkers, which means the risk of subjects with 3/3 > those with 2/3 > those with 1/3 > those with 0/3. Thus the invention relates to determining the risk for an individual subject by assessing the number of markers in the core panel of aneuploidy, p53 and Cyclin A which are abnormal in the subject's sample, and attributing a risk value on the basis of that number of biomarkers which are abnormal.

It may be observed that aneuploidy, p53 and Cyclin A have each been studied by themselves in the context of Barrett's oesophagus in the prior art. However, it is important to note that these markers, if used on their own (i.e. single markers rather than as a panel), are not good enough to provide a reliable and robust result. It is an advantage of the invention that the panel of three biomarkers indicated is used at the same time, i.e. in a multivariate analysis, and it is this which provides technical benefits of the invention.

It is an advantage of the invention that sampling bias is avoided by targeting the biopsies to AFI patches.

It should be noted that the invention is not concerned with the location of the high grade dysplasia (HGD) and/or esophagal adenocarcinoma (EC) within the oesophagus. It is important to note that suitably biopsies are taken from the AFI patches and analysed as taught herein. However, if at least two biomarkers of the core panel are observed, giving an increased likelihood of high grade dysplasia (HGD) and/or esophagal adenocarcinoma (EC) for that subject, then it does not mean that the high grade dysplasia (HGD) and/or esophagal adenocarcinoma (EC) is confined to the exact location of the AFI patch from which the biopsy was taken. In some embodiments the method of the invention provides the advantage that the information provided by said methods is relevant to presence of high grade dysplasia (HGD) and/or oesophageal adenocarcinoma (EC) somewhere within that subject's oesophagus (and not necessarily confined to, or indeed even present in, the particular AFI patch from which the biopsy was taken). In other words, the methods of the invention are useful in detecting or indicating an increased likelihood of HGD/EC within that subject's oesophagus, which HGD/EC need not necessarily be present within the sample (such as a biopsy) itself.

In view of the fact that the prior art regards AFI targeted sampling to be not good enough to produce reliable indications by grading of the lesions, it is especially surprising that the methods taught herein are so effective when practised on biopsies obtained from the AFI patches.

Without wishing to be bound by theory, one reason the high grade dysplasia (HGD) and/or esophagal adenocarcinoma (EC) need not be in the AFI patch from which the biopsy was taken is the so-called "field effect". Regardless of the particular molecular explanation, it is a benefit of the invention that the readout obtained is for the status of the subject being examined and not only for the specific biopsy or patch which was assayed.

In other words, one of the teachings made by the inventors is that it is advantageous to use AFI as an indication of where to collect the biopsy from - it is analysis of the panel of biomarkers as taught herein which adds to the method the information on outcomes/risk stratification/presence of high grade dysplasia (HGD) and/or esophagal adenocarcinoma (EC) for that subject.

If a subject tests positive according to methods of the invention, i.e. if that subject is determined to have an increased likelihood of high grade dysplasia (HGD) and/or esophagal adenocarcinoma (EC) according to the methods of the invention, then suitably that subject may be treated with endoscopic mucosal resection (EMR) and/or may be treated with radio frequency ablation (RFA). Most suitably the subject is treated with RFA.

It is an advantage of the invention that image cytometry analysis may be used to assay aneuploidy. This is especially advantageous since it allows formalin fixed paraffin embedded (FFPE) samples to be analysed.

**Assays**

Aneuploidy

Aneuploidy may be assessed by any suitable means known in the art. Suitably it is determined by a method suitable for use on FFPE material.

Suitably aneuploidy is assayed by determining the DNA content by image cytometry DNA analysis and inferring from said determination whether aneuploidy is present or not. This has the advantage of being operable on FFPE samples.

More suitably aneuploidy is assayed by flow cytometry DNA analysis and inferring from same whether aneuploidy is present or not.

More suitably aneuploidy is assayed by determining the DNA content by flow cytometry DNA analysis and inferring from said determination whether said DNA content is aneuploid.

p53

p53 suitably means NCBI accession number AAD28535. Suitably this has a sequence of:

```
  1 meepqsdpsv epplsqetfs dlwkllpenn vlsplpsqam ddlmlspddi eqwftedpgp
 61 deaprmpeaa prvapapaap tpaapapaps wplsssvpsq ktyqgsygfr lgflhsgtak
121 svtctyspal nkmfcqlakt cpvqlwvdst pppgtrvram aiykqsqhmt evvrrcphhe
181 rcsdsdglap pqhlirvegn lrveylddrn tfrhsvvvpy eppevgsdct tihynymcns
241 scmggmnrrp iltiitleds sgnllgrnsf evrvcacpgr drrtekenlr kkgephhelp
301 pgstkralpn ntssspqpkk kpldgeyftl qirgrerfem frelnealel kdaqagkepg
```

361 gsrahsshlk skkgqstsrh kklmfktegp dsd

Suitably assaying for p53 comprises determining whether there is abnormal staining for p53. This may be done by any suitable method known in the art. Suitably it is determined by a method suitable for use on FFPE material.

In some embodiments assaying for p53 may comprise immunohistochemical staining of the sample for p53, and scoring the stain as normal or abnormal wherein either a strong, dark stain compared to background stain or an absence of stain compared to background stain is an abnormal stain.

Most suitably P53 expression is scored as either positive, negative or not representative in areas of histological abnormality (i.e. dysplasia), relative to background staining and normal histological areas (i.e. non-dysplastic), which served as an internal control [26].

Cyclin A

Assaying Cyclin A may be done by any suitable method known in the art. Suitably it is determined by a method suitable for use on FFPE material.

Suitably Cyclin A is assayed by immunohistochemical staining of the sample for Cyclin A, determining the percentage of Cyclin A positive epithelial surface cells compared to Cyclin A negative epithelial surface cells, wherein a percentage of 1% or more Cyclin A positive epithelial surface cells is an abnormal stain.

More suitably, e.g. in paraffin embedded biopsies, the epithelial surface was defined as the most superficial layer of columnar cells for the glandular tissues. All the surface cells per biopsy were counted up to a maximum of 600 to determine the frequency of Cyclin A expression. When biopsies presented areas of intestinal metaplasia and dysplasia, both were scored to generate a single value. Only cells with diffuse nuclear staining were considered cyclin-A positive. The immunopositive epithelial cells were expressed as a percentage of the total number of epithelial cells counted. The cut-off for Cyclin A positivity was 1% [32].

Methylation

Suitably assay of Runx3, p16, HPP1 means determination of methylation of said gene(s); suitably determination of hypermethylation of said genes.

Methylation may be determined by any suitable method known in the art such as MSP, pyrosequencing, Methylight assay or other suitable technique. Most suitably methylation is determined as in the examples section.

Most suitably determination of methylation may employ probe such as sequencing primer. Exemplary probe(s) and/or primer(s) are shown in the table below.

| Gene name | RefSeq number | Forward primer | Reverse primer | Probe |
|---|---|---|---|---|
| HPP1 | NM_016192 | GTTATCGTCGTCGTTTTTGTTGTC | GACTTCCGAAAAACACAAAATCG | 6FAM5'-CCGAACAACGAACTACTAAACATCCCGCG-3'TAM |
| RUNX3 | NM_004350 | GGGTTTTGGCGAGTAGTGGTC | ACGACCGACGCGAACG | 6FAM5'-CGTTTTGAGGTTCGGGTTTCGTCGTT-3'TAM |
| p16 | NM_000077 | TGGAATTTTCGGTTGATTGGTT | AACAACGTCCGCACCTCCT | 6FAM5'-ACCCGACCCCGAACCGCG-3'TAM |
| ACTB | NM_001101 | TGGTGATGGAGGAGGTTTAGTAAGT | AACCAATAAAACCTACTCCTCCCTTAA | 6FAM5'-ACCACCACCCAACACACAATAACAAACACA-3'TAM |

EP 2 850 208 B1

10

ACTB means beta actin. This is a standard control well known in the art. Suitably this control is assayed in parallel, or at the same time as, or previously or subsequently to, assay of the sample. ACTB is assumed not to be methylated and is suitably used for normalisation of the samples/signal. ACTB is not part of the panel of biomarkers of the invention but may advantageously be assayed for control and/or normalisation purposes.

The skilled worker can look up the known gene sequences from the gene names provided if necessary for the assay. The reference sequences provided in the table above are in order to assist the skilled worker in identifying the genes of interest in case any further guidance is required.

As the skilled person knows, the accession numbers above are Genbank/RefSeq accession numbers. The database entries can be amended over time. Suitably the current database entry is used.

Suitably the database for reference sequences is GenBank (National Center for Biotechnology Information, U.S. National Library of Medicine 8600 Rockville Pike, Bethesda MD, 20894 USA) and accession numbers provided relate to this unless otherwise apparent.

Suitably the database release referred to is 15 April 2013, NCBI-GenBank Release 195.0.

**Dysplasia and Adenocarcinoma**

The invention is primarily concerned with the detection or prediction of high grade dysplasia (HGD) and/or esophagal adenocarcinoma (EC).

The methods of the invention may be applied to aid the diagnosis of high grade dysplasia (HGD) and/or esophagal adenocarcinoma (EC) in a subject.

Thus in one aspect the invention relates to a method of aiding the diagnosis of high grade dysplasia (HGD) and/or esophagal adenocarcinoma (EC) in a subject, the method comprising:

a) providing a sample from an AFI patch of said subject's oesophagus
b) assaying said sample for

(i) aneuploidy
(ii) p53
(iii) Cyclin A

c) wherein if said sample shows the presence of at least two of (i), (ii) and (iii), then said subject is determined as having an increased likelihood of high grade dysplasia (HGD) or esophagal adenocarcinoma (EC).

The methods of the invention are designed to provide information useful in assessing the likelihood of presence of high grade dysplasia (HGD) and/or esophagal adenocarcinoma (EC).

Thus in one aspect the invention relates to a method of collecting information useful in the assessment of the likelihood of a subject having high grade dysplasia (HGD) and/or esophagal adenocarcinoma (EC), the method comprising:

a) providing a sample from an AFI patch of said subject's oesophagus
b) assaying said sample for

(i) aneuploidy
(ii) p53
(iii) Cyclin A

c) wherein if said sample shows the presence of at least two of (i), (ii) and (iii), then said subject is determined as having an increased likelihood of high grade dysplasia (HGD) or esophagal adenocarcinoma (EC).

**Further Benefits of the Invention**

Without wishing to be bound by theory, it is believed that AFI positive areas can be molecularly abnormal. Thus, the invention focuses the analysis on those areas most likely to be abnormal.

Targeting of biopsies by AFI as taught herein can increase the yield of biomarkers.

Most importantly the combination of AFI targeted biopsies together with the analysis of the panel of biomarkers taught herein allows a more objective risk stratification.

It is an advantage of the invention that the three biomarker panels disclosed herein combined with (i.e. analysed on) AFI targeted biopsies accurately stratifies patients based on their prevalent dysplasia.

It is a benefit of the invention that the number of biopsies required is significantly reduced during Barrett's surveillance. It is furthermore an important advance that this reduction in the number of biopsies is accomplished without any loss of sensitivity.

**Further Embodiments**

In some embodiments the panel of the invention (i.e. the analysis of the panel of biomarkers of the invention) may be carried out on a sample which is not an AFI sample. These embodiments of the invention may be referred to as 'panel-only' or 'AFI-independent' embodiments. In such an embodiment the invention relates to a method for determining if a subject has an increased likelihood of high grade dysplasia (HGD) or esophagal adenocarcinoma (EC), the method comprising;

a) providing a sample from said subject's oesophagus
b) assaying said sample for

(i) aneuploidy, and
(ii) abnormal p53, and
(iii) abnormal Cyclin A

c) wherein if said sample shows the presence of at least two of (i), (ii) and (iii), then said subject is determined as having an increased likelihood of high grade dysplasia (HGD) or esophagal adenocarcinoma (EC).

In one embodiment assaying said sample comprises extracting nucleic acid such as DNA from said sample. In one embodiment assaying said sample comprises contacting said sample with a manmade tool such as a PCR primer and/or sequencing primer and/or immunohistochemistry (IHC) reagent such as a manmade antibody recognising one or more of the proteins of interest such as p53 and/or Cyclin A.

**Brief Description of the Figures**

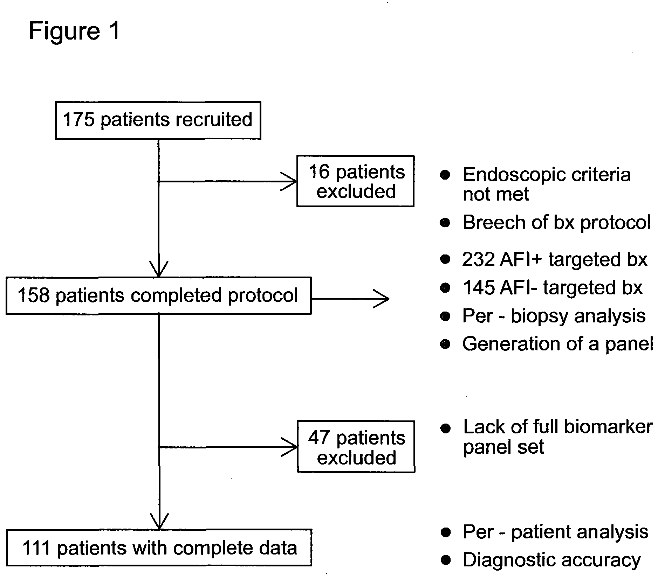

Figure 1 shows a flow chart of recruitment and data analysis.

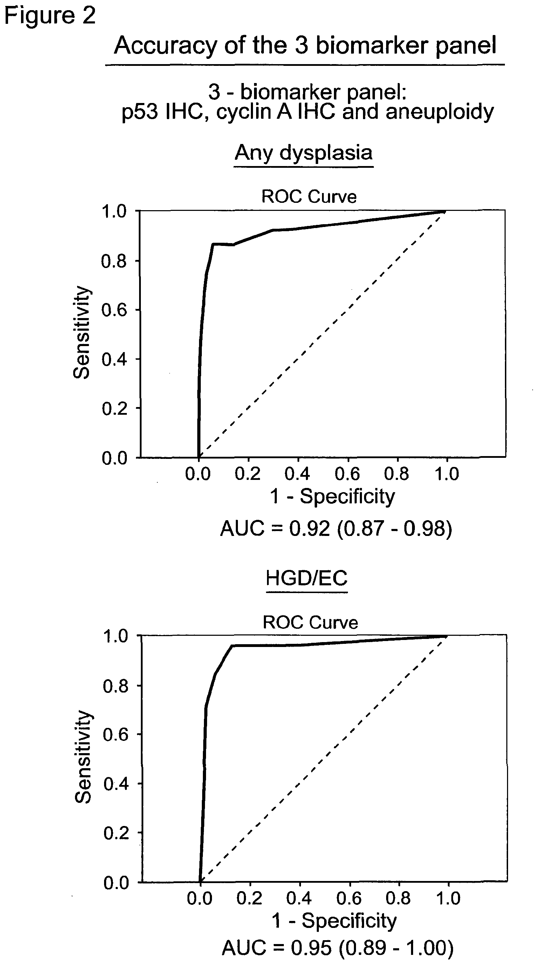

Figure 2 shows graphs illustrating accuracy of the 3-marker panel.

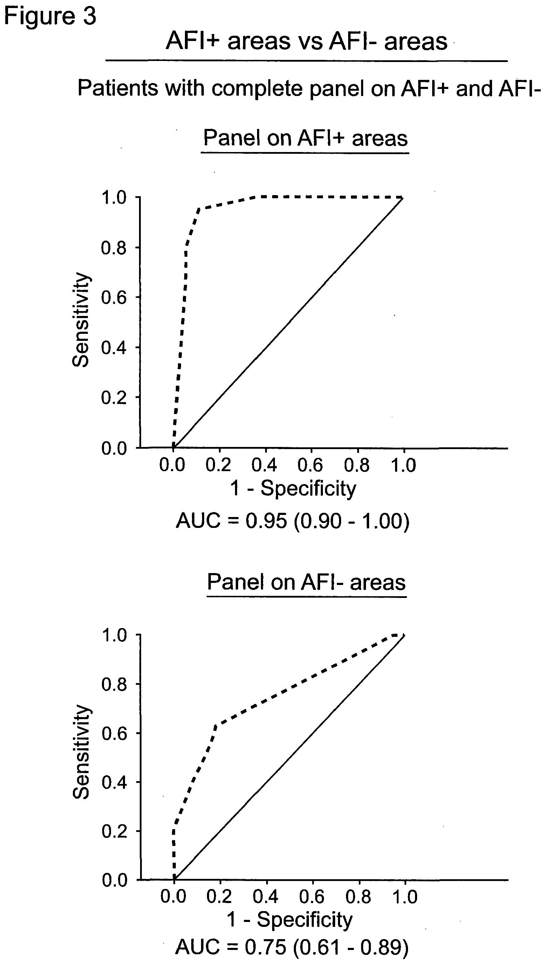

Figure 3 shows graphs illustrating the benefit of AFI+ vs AFI- sampling.

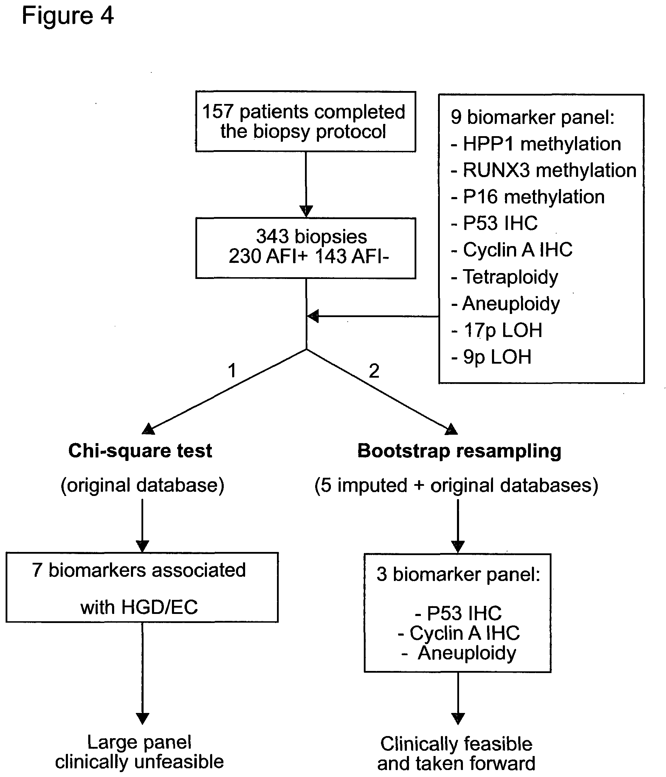

Figure 4 shows a diagram of the strategy for the generation of the biomarker panel. Chi-square test showed that form the initial panel of 9 biomarkers seven significantly associated with a diagnosis of HGD/EC in the corresponding biopsy (arm 1 - left side). To identify a small biomarker panel bootstrap resampling was applied (arm 2 - right side).

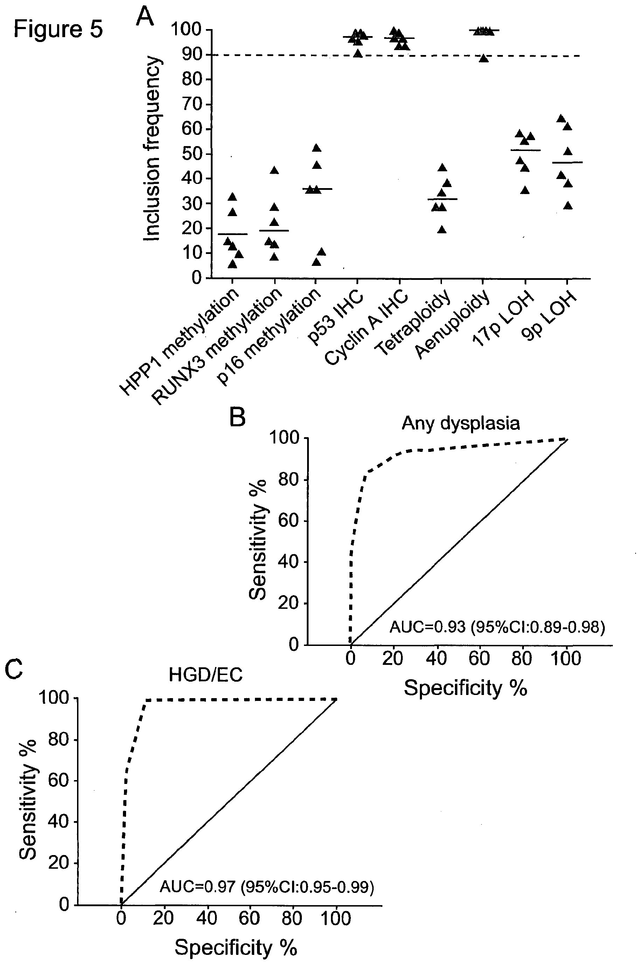

Figure 5 shows a 3-biomaker panel including p53 IHC, cyclin A IHC and aneuploidy has high diagnostic accuracy for dysplasia. A. Inclusion frequencies of the nine biomarkers in 100 bootstrap samples for each MI database (n=5) and the original database (n=1). A stringent cut-off of 90 for the median over all 6 databases was used to select the best biomarkers. P53 IHC, cyclin A IHC and aneuploidy had median inclusion frequency above the threshold. B-C Area under the curve (AUC) for the prediction of any grade of dysplasia (B) and HGD/EC only (C) was calculated using the panel of biomarker selected in A.

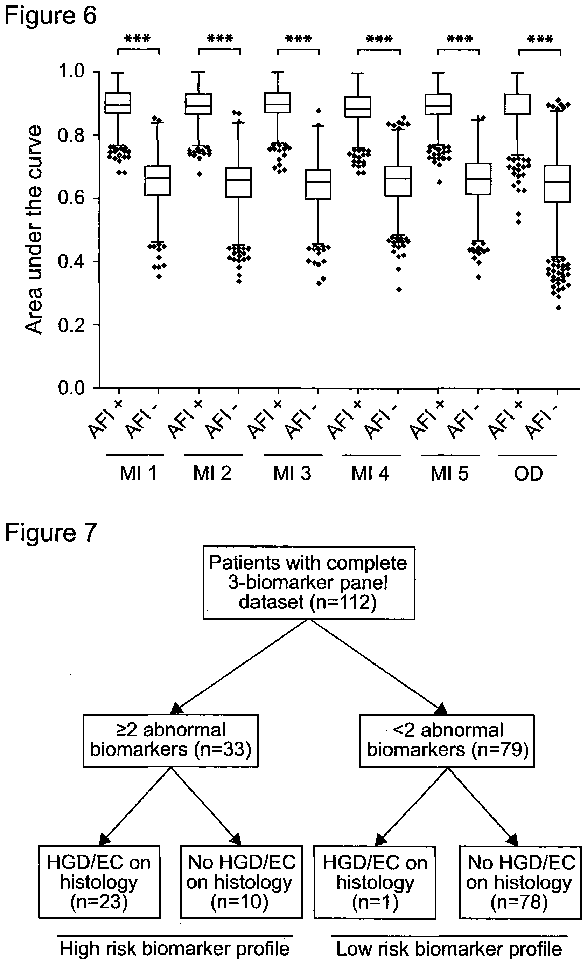

Figure 6 shows diagnostic accuracy for HGD/EC of the 3-biomarker panel assessed on AFI+ areas and AFI- areas. This analysis was performed on 114 patients, after exclusion of patients without AFI positivity (n=35) and those with diffuse AFI positivity (n=8). The AUC for a diagnosis of overall HGD/EC was calculated using the 3-biomaker panel from AFI+ areas and AFI- areas in 2000 bootstrap samples from 5 imputed databases (MI) and the original database (OD). *** indicates p value <0.001
Figure 7 shows a flow-chart of biomarker outcome in patient with full 3-biomaker dataset. Using a cut-off of 2 abnormal biomarkers to diagnose patients with prevalent HGD/EC, the 3-biomaker panel only missed one patients with HGD/EC. Ten patients with NDBE or LGD were classified as high risk for HGD/EC. The sensitivity and specificity of 3-biomaker panel for a diagnosis of HGD/EC were 95.8% (95%CI: 76.9%-99.8%) and 88.6% (95%CI: 79.7%-94.1%), respectively.

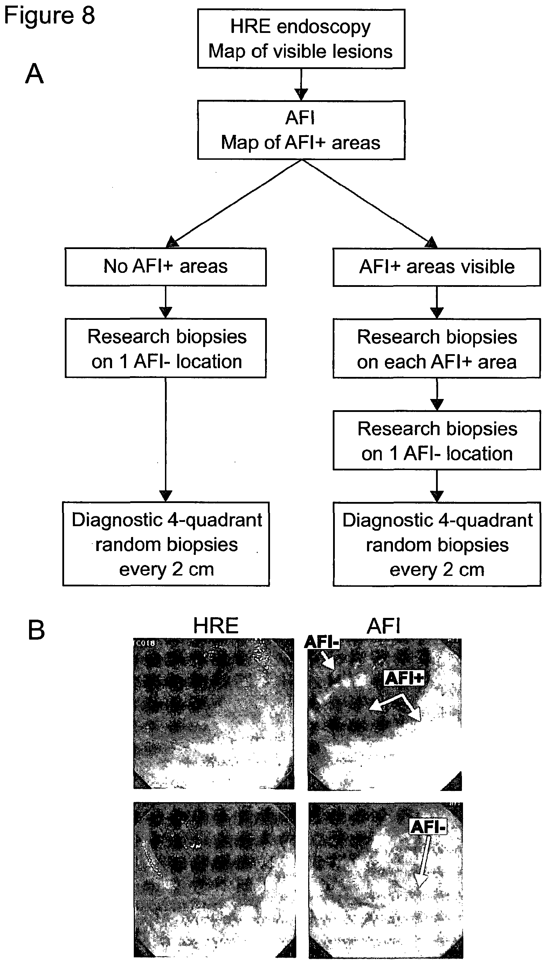

Figure 8 (Supplementary Figure 1) shows endoscopic imaging and biopsy protocols. A. Flowchart of endoscopic imaging and biopsy protocol. B. Examples of endoscopic view on high resolution endoscopy (HRE) and autofluorescence imaging (AFI) in two patients with AFI+ areas (top) and without AFI+ areas (bottom)

The invention is now described by way of example. These examples are intended to be illustrative, and are not intended to limit the appended claims.

**Examples**

**Summary of Examples**

Prospective European multicentre study. Each patient underwent ETMI with targeted biopsies on AFI positive (AFI+) areas and one AFI negative (AFI-) area, as well as random quadrantic biopsies. DNA content abnormalities (aneuploidy/tetraploidy); loss of heterozygosity (LOH) at 9p and 17p loci; RUNX3, HPP1 and p16 methylation; immunohistochemistry (IHC) for p53 and Cyclin A were tested on targeted biopsies. Each biomarker was correlated with the dysplasia and AFI status. Multiple regression analysis was performed to test for independent correlation.

The aims of this study were: a) to validate biomarkers previously published in separate patient cohorts in a single study; b) to assess whether AFI can increase detection of biomarkers; c) to combine ETMI and biomarkers to improve the risk stratification of patients with BO.

Results: a total of 175 patients were recruited, of which 158 generated biopsies for the initial per-biopsy analysis (AFI+, n=231 and AFI-, n=143). Univariate analysis showed that all biomarkers correlated with dysplasia (p<0.05), with exception of 9p LOH. Multivariate analysis showed that aneuploidy, p53 IHC and Cyclin A (3 biomarker panel) were independently associated with dysplasia with an AUC=0.92 (95% CI: 0.87-0.98) for any dysplasia and an AUC=0.95 (95% CI: 0.89-1) for HGD/early cancer (EC). AFI positivity significantly correlated with aneuploidy, p16 methylation, cyclin A and p53 staining (p<0.05). After excluding dysplastic areas, aneuploidy (p= 0.03) and p53 (p= 0.04) staining retained statistical correlation with AFI positivity. Analysis of the 3 biomarker panel in patients with dysplasia showed significant biomarker enrichment in AFI+ compared to AFI- areas (p=0.001). Finally, the 3 biomarker panel was used to predict prevalent dysplasia. Using a cut-off of ≥ 2 biomarkers, the panel when applied to AFI+ areas alone, showed a sensitivity and specificity of 95.8% and 88.5% respectively for the diagnosis of HGD/EC, and 64% and 96% respectively for diagnosis of any dysplasia, compared to overall histology.

Thus we show that AFI increases detection rate for molecular biomarkers. A panel of 3 molecular biomarkers on a small number of AFI targeted biopsies can efficiently predict the dysplasia status and potentially inform therapeutic management of patients with BE.

We show that advanced imaging modalities combined with a new panel of biomarkers can improve the detection of molecular biomarkers in BE.

Hence, our aim is to

1) To prospectively validate biomarkers previously published in separate patient cohorts in a single study
2) To assess whether AFI can increase detection of biomarkers
3) To combine AFI and biomarkers to improve the risk stratification of patients with BE

**Methods**

Patients and setting

This was a prospectively study, conducted in three tertiary referral centres with specialized interest in BE. The study was ethically approved by Cambridgeshire 2 Research Ethics Committee (UK)

Consecutive patients aged > 18 years were recruited, who were either undergoing endoscopic surveillance or follow up post-endotherapy or were referred for work up of dysplasia to the participating centres. Patient were included if they had circumfrential BE segment ≥ 2cm (Prague ≥C2) or non-circumfrential segment of ≥4cm (Prague ≥M4). Shorter segments were excluded as the added value of AFI in very short segment of BE is small due well known AFI-false positivity around the gastro-esophageal junction. Exclusion criteria were: 1. moderate or severe oesophagitis (grade ≥ B, Los Angeles classification); 2. Previous upper GI tract or upper respiratory tract surgery (with exception of anti-reflux Nissen fundoplication) or known upper GI tract abnormality (e.g. pharyngeal pouch); 3. Coagulopathy or anticoagulant therapy (warfarin or clopidogrel) for high risk conditions; 4. Active or severe cardiopulmonary disease or liver disease; 5. Active GI bleeding; 6. Dysphagia; 7. special communication needs.

Endoscopic procedure

After written consent, patient received an endoscopic procedure with ETMI endoscope (XGIF-Q240/260FZ, Olympus inc, Tokyo, Japan)as described before [24]. All the endoscopists (MKS, MDP, JB, KR and ET) had wide expertise in ETMI and performed at least 30 imaging procedures with this technology prior to the study. Briefly patients were sedated with 3-8mg of Midazolam +/- Fentanyl 25-100 mcg or Pethidine 25-50 mg and the esophagus was cleaned with 50% acetylcysteine to disperse any mucus. Simethicone was used at the discretion of the endoscopistfor further cleaning of the esophageal mucosa. First the oesophagus was inspected with white light (WLE) to define the length of the Barrett's segments according to the Prague (C&M) classification and detect any visible macroscopic lesions. Macroscopic lesions, if present, were precisely mapped (level from incisors and quadrant affected). An overview image of the Barrett's segment was taken, plus detailed images of any macroscopically visible lesions. Imaging was then switched to AFI. Positive areas (violet-purple in colour) were carefully mapped. AFI appearance of lesions detected by WLE was also evaluated. An overview picture of the Barrett's segment in AFI mode was taken, plus detailed images of any positive areas. A representative AFI negative area with no suspicious autofluorescence (green colour) or macroscopical abnormality on WLE was randomly selected as negative control. AFI positive areas and negative control area were then inspected with NBI without and with magnification for the assessment of the mucosal and vascular pattern according to previosu classification. All the procedures at the three centres were performed at the presence of two endoscopists (MKS and MDP in Cambridge; KR and ET in Nottingham) or at the presence of one endoscopist plus one fellow with wide expertise in image interpretation (JB and DB in Amsterdam) and the interpretation of the imaging pattern resulted from consensus between the two investigators. Preliminary analysis of the interobserver analysis among centres showed an appropriate K value.

Biopsy and histology

Each AFI positive area (maximum four for each patient) and one AFI negative area received biopsies for biomarker analysis. Where possible (depending on the size of the AFI positive area), the targeted areas received 3 biopsies in the following order of priority: 1 biopsies in formalin, 1 snap frozen biopsy in 10% dimethy sulfoxide (DMSO), 1 biopsy snap frozen dry. Macroscopic lesions were also biopsied independently of their AFI appearance. Finally 4-quadrant biopsies were taken. The histology was assessed by an expert gastrointestinal pathologist at the respective participating centre according to Vienna classification of gastrointestinal neoplasia. All dysplastic cases were then reviewed by a pathologist from one of the other centre. In case of disagreement the third pathologist reviewed the controversial cases to reach majority in the histological call. The highest grade of dysplasia on the combined histology of quadrantic biopsies and macroscopically abnormal area on WLE was considered the "gold standard" in the per-patient analysis

Flow cytometry

All molecular analyses were done at the Medical Research Council/Hutchison Cancer Cell Unit, Cambridge, United Kingdom. the panel of molecular biomarkers analyzed included aneuploidy, G2/tetraploidy, hypermethylation of p16, RUNX3, HPP1 and IHC of p53 and cyclin A.

The snap frozen biopsies in DMSO were used for analysis of DNA content abnormalities and processed as described by Reid et al (gastro 1987). The whole biopsy was sheared and dissociated by mincing and syringing through a small needle in saline buffer [146 mM NaCl, 10mM Tris Base (pH 7.5), 1 mM CaCl2, 0.5 mM MgSO4, 0.05% Bovine serum albumin (BSA), 21 mM MgCl2,Triton X-100 (VWR International ltd, Poole, UK)] with addition of at 10$\mu$g/ml of 4',6-diamidinao-2'-phenylindole (Roche diagnostics GmbH, Mannheim, Germany). The nuclei thus isolated were analyzed by flow cytometry for cell cycle histogram. The flow cytometry was done on MoFlow (Beckman Coulter) and Influx by expert cytometrist. The cell cycle histogram was analyzed using ModFit LT (Verity Software House, Topsham, ME, USA) by MKS and CI. Discordant results were reviewed jointly to reach an agreement. UV laser was used for excitation around 345 nm for MoFlow and 355 nm for Influx. 460/30 band pass filter was used to register emission on MoFlow and 460/50 on Influx. The nuclei distribution was looked at on forward vs side scatter histogram. To exclude aggregates, gating was used on forward scatter vs pulse width histogram. To make sure all clumps were excluded, gating was done on dapi channel vs pulse width plot. To see peaks from cell cycle histogram for Dapi channel (460/50 band pass) in integral/linear scale on MoFlo and in linear scale on Influx gated on Forward/Side scatter population plus singlets discrimination (Pulse width plots) was used.

DNA extraction

DNA was extracted from snap frozen esophageal biopsies using standard phenolchloroform protocol or using the DNeasy® Blood & Tissue Kit (Qiagen, Hilden, Germany) according manufacturers'instructions. Briefly the biopsies were lysed overnight at 50°C in a 180 $\mu$l solution (90 $\mu$l 2X DNA lysis buffer (20 mM Tris/HCl at pH 8, 300 mM NaCl, 4 mM EDTA pH 8, 1%SDS), 90 $\mu$l 1X PBS solution and 20 $\mu$l 20 mg/ml proteinase K (Sigma).. An equal volume of phenol-CIA (chloroform:isoamyl alcohol = 24:1, Invitrogen) was then added, mixed and centrifuged at 4°C to separate the phases (eg Phase Lock Gels). The upper phase was again mixed with an equal volume of phenol-CIA and the process was repeated twice and finally with an equal volume of CIA only. The upper phase was then mixed with 300Mm sodium acetate NaOAc along with 2.5 volumes of 100% ethanol and centrifuged 13,000 rpm for 15 min. The pellet was washed with 1 ml of 70% ethanol and dissolved in TE buffer (10mM Tris, 1 mM EDTA, pH 8).

Bisulfite treatment and quantitative methylation-specific PCR (qMSP)

After bisulfate modification was carried out using EpiTect® Bisulfite Kit (Qiagen, Hilden, Germany) according to manufacturer's instructions, methylation at the CpG islands was assessed by quantitative methylation specific PCR (Methylight) using LightCycler® 480 (Roche diagnostics Ltd, Rotkreuz, Switzerland) for the amplification. Normalized methylation values were calculated as described before [30].

Immunohistochemistry (IHC)

Five 7-micron slides were cut and mounted on glass slides. Staining was performed with BOND™ System (Leica Microsystems, Ltd, Milton Keynes, UK). For both p53 (dilution 1:50, clone DO7, Dakocytomation) and Cyclin A (dilution 1:40, Dakocytomation), the H1 antigen retrieval program was used according to the manufacturers' instructions. A negative control was done by omission of the primary antibody.

The scoring of immunostaining was done by PL-S, EW and MDP who had no prior knowledge of the clinical diagnosis. In paraffin embedded biopsies, the epithelial surface was defined as the most superficial layer of columnar cells for the glandular tissues. All the surface cells per biopsy were counted up to a maximum of 600 to determine the frequency of Cyclin A expression. When biopsies presented areas of intestinal metaplasia and dysplasia, both were scored to generate a single value. Only cells with diffuse nuclear staining were considered cyclin-A positive. The immunopositive epithelial cells were expressed as a percentage of the total number of epithelial cells counted. The cut-off for Cyclin A positivity was 1% [32].

P53 expression was scored as either positive, negative or not representative in areas of histological abnormality (i.e. dysplasia), relative to background staining and normal histological areas (i.e. non-dysplastic), which served as a internal control[26].

Statistics

Categorical variables were described as number and percentage, while continuous variables were described as mean and range. To compare the difference between groups, t-test was used for continuous variables, and Chi-square test was used for categorical variables. A P<0.05 was considered statistically significant. After univariate analysis, all variables that reach the significance level were proposed in the multivariable logistic regression analysis. Those variables that remained significant in the multivariable analysis were selected for the biomarker panel. Receiver operating characteristic (ROC) curves were used to evaluate the distinguishing ability of the biomarker panel and area under the curve (AUC) was reported. We used multiple imputation method to handle the missing data for each biopsy, taking into account variables that significantly associated the target variable in the multivariable logistic regression model. A total of 15 independent imputations were performed. All the analyses were performed using SPSS version 19.0 (SPSS Inc, Chicago, IL, USA).

**Example 1:**

175 participants were recruited (Figure 1), of which 16 were excluded for the following reasons: i. lack of biopsies for laboratory test (n=4); ii. or breach of biopsy protocol (n=12). From the remaining 159 participants, 377 targeted biopsies (AFI+:AFI- = 232:145) were taken and included in the final per-biopsy analysis. Patient demographics are shown in Table 1.

Table 1. Patient demographics and histological stage

| Variables | Number |
|---|---|
| **Sex (M:F)** | **3.73:1** |
| **Age (mean)** | **66.4 (36-87)** |
| **length of Barrett's (mean)** | **7.4 (2-17)** |
| **Histological diagnosis** | |
| **NDBO** | **97 (61.4%)** |

(continued)

| Variables | Number |
|---|---|
| IGD | 23 (14.6%) |
| HGD | 24 (15.2%) |
| EC | 14 (8.9%) |

Since not all the AFI positive areas were large enough to allow the full biopsy protocol; only 111 patients had the minimum dataset required for the inclusion in the final per-patient analysis, which included the three biomarkers forming the panel, as shown below (Figure 1).

The use of AFI within ETMI allowed the identification of 35 additional dysplastic lesions, including 17 HGD/EC lesions, which were missed by WLE. Among the 111 patients, a total 24 cases of HGD/EC were identified by histological analysis on the whole biopsy set available (ETMI targeted plus quadrantic biopsies). AFI targeted biopsies had a diagnostic gain of 3 patients with HGD/EC which would have been otherwise missed by WLE targeted plus random biopsies. However, 4 patients with HGD/EC on random biopsies were missed by targeted biopsies on areas abnormal on any of the three imaging modalities with the ETMI. The sensitivity and specificity for detection of HGD/EC for AFI was 92.7% and 44% and for WLE plus quadrantic biopsies was 89.5% and 82.8% respectively.

Validation of Molecular Biomarkers

In order to assess whether the 9 biomarkers in the initial panel related to the presence of dysplasia in our sample cohort as previously shown in single independent cohorts, we evaluated the association between each biomarker and dysplasia on per biopsy analysis. The size of the AFI area limited the number of biopsies taken and hence the number of biomarkers analyzed in each area differed. On univariate analysis all the molecular biomarkers were significantly associated with any grade of dysplasia (which included LGD, HGD and EC), except 9p LOH. Similarly, with the exception of tetraploidy and 9p LOH, all the other molecular biomarkers were significantly associated with HGD/EC. In the multivariable logistic regression model adjusting for all the biomarkers, Aneuploidy, IHC staining of p53 and Cyclin A remained significantly associated with any grade of dysplasia and HGD/EC (Table 2).

Table 2. Correlation of biomarkers with dysplasia

| | Any dysplasia | | | HGD/EC | | |
|---|---|---|---|---|---|---|
| Biomarker | No. of biopsies | Any dysplasia | Univariate analysis | Multivariate analysis | HGD/EC | Univariate analysis | Multivariate analysis |
| HPP1 methylation | 302 | 71 | 0.004* | 0.22 | 41 | 0.02* | 0.35 |
| RUNX3 methylation | 302 | 71 | <0.001* | 0.19 | 41 | 0.002* | 0.33 |
| P16 methylation | 302 | 71 | 0.001* | 0.67 | 41 | 0.001* | 0.10 |
| P53 IHC | 324 | 79 | <0.001* | <0.001* | 43 | <0.001* | 0.05* |
| Cyclin A IHC | 319 | 71 | <0.001* | <0.001* | 37 | <0.001* | 0.01* |
| Tetraploidy | 302 | 65 | 0.002* | 0.21 | 35 | 0.29 | 0.70 |
| Aneuploidy | 302 | 65 | <0.001* | 0.001* | 35 | <0.001* | <0.001* |
| 17p LOH | 269 | 65 | <0.001* | 0.07 | 38 | <0.001* | 0.49 |
| 9p LOH | 265 | 62 | 0.70 | 0.57 | 37 | 0.73 | 0.67 |

Correlation of molecular biomarkers with AFI status

We then asked whether the 9 biomarkers were associated with the AFI status of the areas identified at endoscopy. Aneuploidy, 17p LOH IHC of p53 and Cyclin A were significantly associated with AFI positivity. Since dysplasia could be a confounding factor, we then analyzed the relationship between biomarkers and AFI positivity, after exclusion of dysplastic lesions. Aneuploidy and IHC p53 staining retained significant association with AFI positivity (Table 3).

Table 3. Correlation of molecular biomarkers with AFI status

|  | All AFI positive areas | | Non dysplastic AH positive areas | |
|---|---|---|---|---|
| Biomarker | No. of biopsies | p value | No. of biopsies | p value |
| HPP1 methylation | 303 | 0.25 | 231 | 0.79 |
| RUNX3 methylation | 303 | 0.12 | 231 | 0.55 |
| P16 methylation | 303 | 0.13 | 231 | 0.85 |
| P53 IHC | 325 | <0.001* | 245 | 0.04* |
| Cyclin A IHC | 319 | 0.02* | 248 | 0.43 |
| Tetraploidy | 303 | 0.46 | 237 | 0.50 |
| Aneuploidy | 303 | <0.001* | 237 | 0.02* |
| 17p LOH | 270 | 0.04* | 204 | 0.17 |
| 9p LOH | 266 | 0.61 | 203 | 0.46 |

To confirm the finding that biomarker positivity correlated with AFI status, we compared the presence of the three biomarkers selected by the multiple logistic regression (aneuploidy, p53 IHC and cyclin A)in AFI positive and AFI negative areas from patients with dysplasia (including LGD, HGD and EC) anywhere in the BE segment. Notably, about 70% of the AFI negative areas harbored one or none of these biomarkers, whereas about 73.6% of the positive areas had at least 2 biomarkers positive. This difference was statistically significant (p = 0.001) (Table 4).

Table 4. Enrichment of molecular biomarkers in AFI positive areas

|  | No. of abnormal biomarkers | | | | |
|---|---|---|---|---|---|
|  | 0 | 1 | 2 | 3 | P value |
| AFI- | 14 (42.4%) | 9 (27.3%) | 7 (21.2%) | 3 (9.1%) | 0.001 |
| AFI+ | 8 (14.3%) | 7 (12.5%) | 21 (37.5%) | 20 (35.7%) |  |

Risk stratification

Since the multiple logistic regression in the per-biopsy analysis indicated that aneuploidy, p53 IHC and cyclin A are independently related to dysplasia and the biomarker enrichment analysis confirmed that the yield of these molecular abnormalities is higher in AFI positive areas, we next considered these three biomarkers as a panel in combination with AFI status to assess their performance in predicting the prevalent dysplasia status of the patients. One hundred and eleven patients were included in this analysis, which represent those patients with all three biomarkers available in the AFI targeted biopsies or those with all three biomarkers available in a random biopsy if no AFI abnormal area was found. Patients were classified as having either no biomarkers positive, or from 1 to 3 abnormal biomarkers (table 5).

Table 5. Diagnostic accuracy of the 3-biomarker panel

|  |  | NDBE | HGD/EC | Total |
|---|---|---|---|---|
| No. of abnormal biomarkers | 0 | 49 (100.0%) | 0(0) | 49 |
|  | 1 | 28 (96.6%) | 1 (4.5%) | 29 |
|  | 2 | 7 (46.7%) | 8 (53.3%) | 15 |
|  | 3 | 3 (16.7%) | 15 (83.3%) | 18 |
| Total |  | 87 | 24 | 111† |

By matching the biomarker status with their histologic diagnosis (presence or absence of HGD/EC) we found that only 1 patient with HGD had less than 2 biomarkers positive. Using 2 biomarkers as cut off for dividing patients in two risk categories; this biomarker panel had a sensitivity of 95.8% and a specificity of 88.5% in predicting prevalent dysplasia. We then compared this diagnostic accuracy, with that of either histology on ETMI targeted biopsies or histology on WLE targeted + 4-quadrant biopsies, which had a sensitivity of 92.1% and 90% respectively and a specificity of 80.5% and 22.8% respectively. In particular we found that the biomarker panel stratification improved the sensitivity of the gold standard approach (WLE targeted + 4-quadrant biopsies), with only 1 HGD/EC patient misdiagnosed , compared with 2 HGD/EC patients downgraded by WLE targeted plus random biopsies, but the difference was not statistically significant (p= 1.0). We further evaluated our biomarker panel in the 15 imputed databases. Among the 156 patients in the analysis, the average number of HGD/EC patient misdiagnosed were 4.47, which was not significantly different from the original database and the results by WLE targeted plus random biopsies.

Number of biopsies

Standard endoscopic surveillance with 4-quadrant biopsies is fraught with the disadvantage of relying on an enormous number of biopsies, especially for long segments of BE, with significant costs for the health systems and risks for the patients. We therefore looked into whether the stratification by biomarkers and AFI could improve these drawbacks. We found that the standard diagnostic approach generated approximately 1700 biopsies in 111 patients with average BO length of 7.6cm, yielding 22 diagnoses of HGD/EC (out of a total of 24 patients with HGD/EC), corresponding to an average number of 42 biopsies for each HGD/EC diagnosed (Table 6).

Table 6. Clinical utility of the novel approach compared to standard endoscopic techniques with histologic analysis

|  | Histology on WIE targeted + 4-Q biopsies | EIMI+ histology | AFI + Biomarkers |
|---|---|---|---|
| Total no. of biopsies | ~1700 | ~250 | ~250 |
| No of biopsies for every HGD/EC case diagnosed | 42 | 7.5 | 6.5 |
| No. of HGD missed | 2 | 3 | 1 |

Conversely, the alternative method for risk stratification only relied on 250 biopsies (18 random biopsies from patients without a AFI abnormal area and 166 AFI positive biopsies) to correctly classify as high risk 23 out of 24 patients, with only 7.5 biopsies needed for each high risk patient identified. The average number of biopsies taken by the standard diagnostic approach was 15.6), while the alternative method for risk stratification only took 1.7 biopsies on average. WLE targeted and random quadrantic biopsies combined generated a total of about 1700 biopsies leading to detection of 35 HGD/EC and misclassification of 3 cases of HDG/EC as non-dysplastic. AFI positive targeted biopsies generated 268 biopsies and detected 37 HGD/EC and misclassified only four cases of HGD/EC. Thus, the number of biopsies needed to diagnose one case of HGD/EC by the later method was significantly lower at 7.4 compared to 50 by the former technique. Among these 7 patients who was missed by either WLE targeted and random quadrantic biopsies or AFI positive targeted biopsies, four was correctly classified as high risk by the AFI directed 3 biomarker panel, two do not have all the 3 biomarkers tested, and only one was misclassified as low risk.

Further data in support is provided in Figure 2 and Figure 3.

Conclusions

This is the first study in patients with BE, to independently evaluate a large number of molecular biomarkers prospectively in a phase 4 study. In addition, this is the first study to use the novel combination of advance imaging and biomarkers to predict prevalent dysplasia and risk stratify BE patients based on molecular biomarkers taken from AFI positive areas on significantly fewer biopsies.

A number of phase 3 studies from different centers found that single biomarkers or panels of biomarkers correlate with dysplasia in BE and can predict the individual risk of progression to HGD/AC [26, 30-32]. However, the lack of validation of these biomarkers in independent patient cohorts has hampered their translation into clinical practice. With this study, we aimed to validate a large panel of 9 biomarkers from afore mentioned studies in a large prospective phase 4 study. Our results confirmed previous findings, in that the majority of the biomarkers tested significantly correlated with dysplasia at univariate analysis. Clearly, there is a need for making the use of biomarkers clinically applicable in terms of number of tests needed to predict outcome, feasibility in routine clinical setting and costs for the health system. After logistic regression analysis we found that three of these biomarkers (aneuploidy, p53 IHC and cyclin A) correlated independently with dysplasia. These three biomarkers can represent a feasible panel, since two of them are immunohistochemical markers, of which p53 is already routinely used in many pathology laboratories to aid diagnosis of dysplasia, whereas cyclin A has a simple read out based percentage of cyclin A expressing cells on the surface. With concern to aneuploidy, we employed a published method based on FACS analysis of a cell population isolated from frozen samples [31]. This is clearly a more complex procedure, however it was important to use a published method to avoid that inconsistent results with previous studies could have grounded on different methodology. However, aneuploidy can be also assessed successfully with image cytometry on paraffin section and immunoistochemical markers acting as surrogate of aneuploidy are usable. Therefore we believe that this biomarker panel is clinically feasible.

When analyzing biomarkers on tissue samples from BE segments, one of the key questions is how much one or very few biopsies are representative of the entire BE segment. There is evidence that the Barrett's metaplasia can be highly heterogeneous with regards to the molecular defects [33, 34] and therefore, similarly to dysplasia, which can be often inconspicuous, targeting biopsies to areas of molecular change is difficult at standard imaging. In the recent years, large effort has been put at investigating novel imaging modalities to identify inconspicuous neoplasia. AFI has shown promising results, but its applicability in clinical practice has been limited by a very high false positive rate (up to 80%) and despite similar sensitivity to the standard protocol, published data do not suggest that AFI can replace the standard biopsy protocol in the prior art. Since AFI positivity however is related to molecular, as well as architectural changes, we hypothesized that false positivity at AFI can aid the detection of molecular biomarkers. DNA is known to be a poor fluorophore and gross DNA abnormalities, which can precede development of dysplasia, could influence the AFI appearance of BE. Indeed, we found that aneuploidy and p53 abnormalities are significantly associated with AFI positivity independently from the presence of dysplasia. While this is intuitive for aneuploidy, it can also be explained for p53, since this genome guardian protein is implicated in the control of DNA amplification[35]and proliferation of aneuploid cells [36]. Furthermore, we found a significant enrichment of the 3-biomarker panel in AFI positive areas compared to AFI negative areas in dysplastic patients. In keeping with this, none of the patients with an entirely AFI negative BE had a high risk profile on biomarker analysis nor had dysplasia. Altogether these data show that AFI is an effective tool to direct biopsies for biomarkers analysis and reduce the number of biopsies needed for risk stratification.

The combination of AFI and biomakrers proved to be the best stratification tool in our per-patient analysis. Our data on the performance of ETMI in detecting dysplasia are in line with recent findings that despite high sensitivity figures, ETMI with histology on targeted biopsies cannot replace the standard protocol and suffers from low specificity[24, 25]. Also in our study, ETMI targeted biopsies would have misdiagnosed four patients with HGD/EC as non-dysplastic or LGD. On the other hand, the standard surveillance protocol proved to be imperfect too, since was fraught with misdiagnosis of 3 patients, despite extensive biopsies. The new stratification tool of the invention showed the highest sensitivity and specificity figures, and it is remarkable that only one HGD patient fell out of the high risk category.

One of the major criticisms reserved to the standard biopsy protocol, is the very high number of biopsies needed especially for long segment of BE, with subsequently increased risk of bleeding for the patients, lengthy endoscopic procedures and high work-load for pathology laboratory. The combination of a flagging technique such as AFI and biomarkers can potentially overcome this important limitation of the endoscopic surveillance. In our study, the alternative protocol was not inferior to the standard one, but allowed to reduce by 7-fold the total number of biopsies. This would certainly limit risks and discomfort to the patients. There is also the potential to limit costs for the healthcare, provided that more feasible techniques for assessment of aneuploidy as discussed above can be used.

From a strict scientific perspective it is important to objectively note that, as with all analyses of this type, this study has a number of limitations. First, there are no follow up data on the patient cohort. In fact, this study was designed to assess for the ability of biomarkers to predict prevalent dysplasia, and not to stratify patient according to their risk of progression. The specificity of the 3-biomarker panel was only 88%, due to the fact that 11 patients had a high risk biomarker profile, despite not having evidence of HGD/EC. However follow up on these patients showed that that 5 of them developed HGD/EC at follow up endoscopies, 3 had persistent LGD and remaining 3 are still awaiting follow-up. Among the patients with LGD 2 had RFA treatment with complete pathological response and therefore their outcome cannot be assessed. This limited follow up data do show that this panel has the potential to also risk stratify patient according to their future risk, however future studies will need to confirm this. Second, only approximately 2/3 of participants could enter the per-patient validation analysis. This is due to the fact that at start we lacked information about which of the 9 biomarkers was going to outperform the others in the multivariate analysis. Therefore, since not all the AFI positive areas were large enough to allow 3 research biopsies we made all the efforts to evenly distribute biomarker analyses among the available biopsies. This inevitably resulted in some patients not having the minimum dataset required to enter the second phase analysis. However, we believe that the final number of patients that informed the analysis on the diagnostic accuracy was large enough to reach robust conclusions. Third, the assessment of the dysplasia on the research frozen biopsies relied on the FFPE sample taken in close vicinity rather than on the frozen biopsy itself, therefore there is a possibility that very focal dysplasia on frozen samples could have been missed. The endoscopist however were all very experienced in BE surveillance and made all the efforts to take research biopsies all in close proximity. Fourth, this patient cohort was enriched for dysplasia, therefore it does not fully reflect the general BE population. However, enrichment for high risk patients was necessary to guarantee an adequate number of pathologic outcomes to correlate with biomarker positivity.

For all these reasons, the data presented herein can be seen to provide a robust and positive demonstration of working of the invention, and serve to illustrate the utility and numerous technical benefits provided. In addition, the efficacy of the invention compared to the shortfall in the prior art techniques is clearly shown.

## Example 2

We demonstrate the combination of autofluorescence endoscopy and molecular biomarkers is a novel diagnostic tool for dysplasia in Barrett's esophagus.

BACKGROUND & AIMS: Endoscopic surveillance for Barrett's esophagus (BE) has major limitations including multiple biopsies, sampling error and subjectivity of pathological diagnosis of dysplasia. We hypothesized that molecular biomarkers on biopsies targeted by an endoscopic red-flag technique could overcome these limitations. We aimed to compare a biomarker panel on minimal biopsies directed by autofluorescence imaging (AFI) with the standard surveillance protocol for the diagnosis of prevalent dysplasia.

METHODS: We performed a cross-sectional prospective study on 175 patients with BE in three tertiary referral centers. All patients underwent high resolution endoscopy followed by AFI. Aneuploidy/tetraploidy; 9p and 17p loss of heterozygosity; *RUNX3, HPP1* and *p16* methylation; p53 and cyclin A immunohistochemistry (IHC) were assessed on biopsies targeted by AFI. Bootstrap resampling was used to select the best predictive biomarker panel for high grade dysplasia (HGD) and early cancer (EC).

RESULTS: Aneuploidy, p53 IHC and cyclin A had the strongest association with dysplasia in the per-biopsy analysis and, as a panel, had an area under the receiver operating characteristic curve of 0.97 (95%CI 0.95-0.99) for the diagnosis of HGD/EC. Biomarkers were enriched in AFI+ compared to AFI- negative areas and the diagnostic accuracy for HGD/EC of the 3-biomarker panel form AFI+ areas was superior to AFI- areas (p<0.001). Furthermore, this 3-biomarker panel had equal sensitivity to the standard protocol for HGD/EC with a 4.5-fold reduction in the number of biopsies.

CONCLUSIONS: A 3-biomarker panel on a small number of AFI-targeted biopsies provides an accurate and objective diagnosis of dysplasia in BE.

## Background

The incidence of esophageal adenocarcinoma (EA) has dramatically increased over the last 40 years in the Western world and it is expected to rise further. Survival rates for EA remain dismal with only 14% patients alive at 5 years from diagnosis. [2, 3] Barrett's esophagus (BE) is the only recognized precursor to EA and progresses to invasive cancer through intermediate dysplastic stages, namely low grade dysplasia (LGD), high grade dysplasia (HGD) and early intramucosal cancer (EC). [4] Diagnosis of EA in patients with known BE is associated with improved survival, [5] therefore specialist societies recommend endoscopic surveillance for patients with BE using a protocol entailing four-quadrant random biopsies every two centimeters plus targeted biopsies of visible lesions (Seattle protocol). [6, 7] Diagnosis of HGD or EC leads to treatment recommendations generally involving endoscopic therapy to prevent development of advanced disease. [8] However, endoscopic surveillance has major limitations. The Seattle protocol is time consuming and invasive with subsequent poor adherence by endoscopists. [9, 10] In addition dysplasia and EC can be inconspicuous at endoscopy leading to significant sampling error by random biopsies. [11] Hence, clinical justification and cost-effectiveness of endoscopic surveillance has been questioned, especially following recent evidence that the cancer risk in BE may be lower than previously thought. [12,13]

To improve recognition of inconspicuous dysplasia, autofluorescence imaging (AFI) has been investigated. [14-16] AFI utilizes short wave-lengths of light to excite certain endogenous molecules (fluorophores) in the gastro-intestinal (GI) mucosa to emit a fluorescent signal. Dysplasia and EC in the esophagus can be associated with loss of autofluorescence, due to distortion of tissue architecture and molecular changes. [16] AFI can improve detection of inconspicuous dysplasia, however it suffers from a high false positive rate (>60%). Inflammation has been proposed to explain this false positivity, however this has not been proven. We hypothesized that the AFI positivity may correlate with molecular abnormalities of the glandular tissue, not yet manifest as histological dysplasia.

An additional limitation of the current surveillance strategy is that the histopathological diagnosis of dysplasia is fraught with significant inter-observer variability even among expert pathologists. [17, 18] To overcome this, researchers have investigated molecular biomarkers to assess disease stage in a more objective way. Some of these, such as over-expression of p53 [19-21] and cyclin A, [22] methylation of specific genes, [23] loss of heterozygosity (LOH) at the 17p and 9p loci and DNA ploidy abnormalities [24] have been showed to associate with dysplasia. However, with the exception of p53 expression, these biomarkers have only been tested by single groups in single cohorts of patients and this has hampered their translation into clinical practice. A limitation of biomarkers on random biopsies is that, similarly to dysplasia, they are potentially subject to sampling error, due to the non-uniform distribution of molecular changes in BE. [24, 25] This limitation could be overcome using advanced imaging modalities to help target biopsies for biomarkers. We assessed the feasibility of this novel approach in a recent retrospective study 26

The inventors hypothesised that combining AFI with molecular biomarkers could provide a synergistic approach to the assessment of the dysplasia status of the patient. Therefore, the primary aim of this prospective study was to compare the accuracy of a panel of molecular biomarkers on AFI-directed biopsies with the current gold standard (Seattle protocol) for the diagnosis of HGD/EC in BE. A secondary aim of this study was to determine whether previously validated biomarkers could be replicated in an independent prospective study in which the biomarker evaluation was performed in an independent laboratory.

**Methods**

Patients and setting

The study was approved by the Cambridgeshire 2 Research Ethics Committee (09/H0308/118). Patients aged > 18 years were recruited prospectively from three centers. Inclusion criteria were: endoscopic surveillance for BE, referral for evaluation of dysplasia or follow-up post endoscopic resection for HGD/EC. In addition all patients had to have circumferential BE ≥ 2cm (Prague ≥C2) or tongues ≥4cm (Prague ≥M4). Shorter segments were excluded due to the excess of AFI-false positivity at the gastro-oesophageal junction. [27] Exclusion criteria were: moderate or severe esophagitis; previous upper GI surgery (with exception of Nissen fundoplication) or known upper-GI tract abnormality (e.g. pharyngeal pouch); coagulopathy or anticoagulant/antiplatelet therapy for high risk conditions; active or severe cardiopulmonary disease or liver disease; dysphagia; special communication needs.

Endoscopic procedure

After written informed consent, patients were endoscoped with a XGIF-Q240 or FQ260Z endoscopes (Olympus inc, Tokyo, Japan) as previously described. [14] All the endoscopists had performed at least 30 AFI procedures prior to the study. The esophagus was inspected first with white light high resolution endoscopy (HRE) to define the length of the BE segment as per the Prague classification and to record the presence of any macroscopically visible lesions (Figure 8 (Supplementary Figure 1A)). After switching to the AFI mode, AFI positive (AFI+) areas (violet-purple in color) were carefully mapped. A representative AFI negative (AFI-) area (green color) was then selected as a negative control (Figure 8 (Supplementary Figure 1B)). In patients who had diffuse patchy AFI positivity throughout the BE (n=8) the AFI negative control area was not selected.

Biopsy and histology

Each AFI+ area (maximum of four for each patient) and one AFI- area were biopsied for biomarker analysis and histopathology (Figure 8 (Supplementary Figure 1A)). Where possible (depending on the size of the AFI+ area), a maximum of three biopsies were taken in the following order of priority: one biopsy in formalin, one snap frozen biopsy in 10% dimethysulfoxide (DMSO), one biopsy snap frozen dry. Histopathological assessment of each AFI-targeted area relied on the biopsy stored in formalin. Random biopsies were then taken according to the Seattle protocol. The histology was assessed by an expert GI pathologist at the respective participating center according to the Vienna classification. [28] All dysplastic cases, including indefinite for dysplasia (ID) were then reviewed by a second pathologist from one of the other centers. In case of disagreement, consensus among all pathologists was reached through a review process. Cases with ID which were not up-graded to higher stages after consensus review were regarded as non-dysplastic (NDBE). In accordance with the Vienna classification, for the purpose of this paper, cases of HGD, carcinoma in situ and intramucosal adenocarcinoma were grouped together (HGD/EC). These histological entities represent a common endpoint for endoscopic therapeutic intervention according to clinical guidelines [6] and expert consensus statements. [29] For the purpose of the biomarker analysis, all biopsies taken using the AFI mode were regarded as AFI-targeted (AFI+ and AFI-). With regards to the overall per-patient histopathological diagnosis, we considered diagnoses made using: a) the standard protocol (biopsies on areas visible on HRE + random biopsies); b) the overall histology (a + histology on AFI-targeted biopsies).

Biomarker analyses

All molecular analyses were done at the Medical Research Council (Cambridge, UK). The panel of molecular biomarkers analyzed comprised: aneuploidy, G2/tetraploidy, LOH at 9p and 17p loci, hypermethylation of *p16*, *RUNX3*, *HPP1* and immunohistochemistry for p53 and cyclin A.

Flow cytometry

The snap frozen biopsies in DMSO were used for analysis of DNA content abnormalities and processed as described previously. [30] The isolated nuclei were analyzed by flow cytometry using a MoFlow (Beckman Coulter, Miami, FL, USA) or BD Influx™ (Becton, Dickenson biosciences, New Jersey, USA) by an expert cytometrist. The cell cycle histogram was analyzed using ModFit LT (Verity Software House, Topsham, ME, USA) by two investigators and discordant results were reviewed jointly to reach agreement.

Quantitative methylation-specific PCR (Methylight)

DNA was extracted from snap frozen esophageal biopsies using the DNeasy® Blood & Tissue Kit (Qiagen, Hilden, Germany) according to the manufacturer's instructions. DNA was bisulfite modified using an EpiTect® Bisulfite Kit (Qiagen) and the degree of methylation was analyzed by Methylight using a LightCycler® 480 (Roche diagnostics Ltd, Rotkreuz, Switzerland) with previously published Taqman primers and probes. [23] Normalized methylation values were calculated as previously described. [23]

Immunohistochemistry (IHC)

Staining was performed with the BOND™ System (Leica Microsystems, Ltd, Milton Keynes, UK) with an H1 antigen retrieval program. Antibodies used were: p53 clone DO7 (Dakocytomation, 1:50) and cyclin A (Novocastra, 1:40). The scoring was performed without prior knowledge of the clinical diagnosis. Surface cyclin A was scored as previously described, using a cut-off for positivity of 1% of positive surface cells. [22] P53 expression was scored positive when there were areas of strong staining or complete loss of staining, compared to the background levels, as previously described. [31]

Loss of heterozygosity (LOH)

LOH at 17p and 9p loci (*p53* and *p16* genes, respectively) was assessed on DNA extracted by phenol/chloroform. Microsatellite markers were used as previously published. [32] The microsatellite loci were amplified by multiplex PCR and the PCR products were combined with GeneScan™ LIZ® Size Standard and electrophoresed on an automated sequencing system (ABI 3130 xl Genetic Analyzer, Applied Biosystems, California, USA) according to the manufacturer's protocol. GeneMapper® Software (Applied Biosystems) was used to analyse the peak height ratios of the alleles of a duodenal control and oesophageal biopsy and highlight LOH candidates. LOH was assessed on the basis of the following allelic imbalance ratios:

$$\text{Allelic ratio of sample} = \frac{\text{Peak height of Allele 1}}{\text{Peak height of Allele 2}}$$

$$\text{Allelic Imbalance} = \frac{\text{Allele Ratio of Control Sample}}{\text{Allele Ratio of AFI Sample}}$$

Ratio of > 1.35 or < 0.067 was considered as suggestive of LOH as instructed by the software. The sample was considered positive for LOH if ≥2 loci were suggestive for LOH for a particular gene.

Statistics

For the purpose of the per-biopsy analysis, each AFI-targeted area was classified with: *i.* patient research number; *ii.* AFI status (AFI+ or AFI-); *iii.* binary outcome of the 9 biomarkers (positive or negative); *iv.* histological diagnosis (NDBE, LGD, HGD, EC). Chi-square tests were used to compare differences between groups. A p-value <0.05 was considered statistically significant. In some AFI-targeted areas some biomarkers were recorded as missing. To ensure enough power, missing data were imputed by multiple imputation (MI). [33] The imputation model included the 9 biomarkers and the histological diagnosis variable. Five independent datasets were imputed. The original database was used after removing samples containing missing values in variables of interest.

In order to identify a biomarker panel that could predict histological outcome, we used a bootstrap resampling

method [34] and took 100 bootstrap samples for each MI dataset and the original database. For each bootstrap sample, we tested individually the 512 different combinations of 9 biomarkers for their association with HGD/EC. Out of the 512 possible models, the best one was selected according to the Akaike information criterion (AIC) of a logistic regression model. In order to correct for potential patient effects when multiple AFI-targeted areas derived from the same patient, they were included as random effects in the model. For each biomarker we calculated the bootstrap inclusion frequency, which was defined as the number of times the biomarker was selected in the 100 best models. The biomarkers with a median inclusion frequency of at least 90 over the imputed datasets and original database were selected for the biomarker panel. [35] The predicted probability of dysplasia for each endoscopic area obtained from a logistic regression with the selected biomarker panel as predictor was used to generate the receiver operating characteristic (ROC) curves to evaluate the diagnostic accuracy of the panel and the area under the ROC curve (AUC) was reported. For the purpose of the per-patient analysis, in patients with at least one AFI+ area and one AFI-area two datasets, called AFI+ and AFI-dataset, were created. In the AFI+ dataset, each biomarker was assigned a positive outcome, if the biomarker was positive in at least one AFI+ area, and negative if negative in all AFI+ areas. An identical process was followed for the AFI-dataset. The histological diagnosis for a patient was the overall histology (see biopsy and histology section). A logistic regression model, with the selected biomarker panel as predictor, was fitted using patients from a bootstrap sample and was then validated in the patients that were not selected in the bootstrap sample. This process was repeated 2000 times separately for the AFI+ and the AFI- datasets, resulting in 2000 AUC values for each dataset, which were compared in a paired t-test pairing bootstrap samples from the AFI+ and AFI- datasets. This comparison was done in all the 5 imputed databases and the original database.

To validate in the per-patient analysis the biomarker panel selected in the per-biopsy analysis, we applied a simple counting approach to the original database. For each possible panel of two or three biomarkers, a patient was predicted as HGD or EC if the number of positive biomarkers in the panel reached a certain cut-off point. We calculated sensitivity and specificity of each panel for a diagnosis for HGD/EC based on different cut-off points. The best biomarker panel was defined as the one with the highest accuracy (average of sensitivity and specificity). This process was repeated in 2000 bootstrap samples from the original database and the mean and standard deviation of the accuracy was reported for each panel at different cut-off points.

All the analyses were performed using SPSS version 19.0 (SPSS Inc, Chicago, IL, USA) and R version 2.15.2.

**Results**

Of the 175 participants recruited, 18 were excluded because of either lack of biopsies for laboratory tests (n=4) or breach of biopsy protocol (e.g. biopsy mislabeling, no research biopsy in AFI+ areas taken, no random biopsies taken) (n=14). From the remaining 157 participants, we obtained 373 AFI-targeted biopsies (AFI+: AFI- = 230:143), which were processed for biomarkers and included in the per-biopsy analysis. Patient demographics and histology outcome are summarized in Table A.

**Table A.** The demographics and histological stage of patients that completed the biopsy protocol (n=157).

| Variables | |
|---|---|
| Male:Female ratio | 3.76:1 |
| Mean age (range) | 66.4 (36-87) |
| Mean length of Barrett's in cm (range) | 7.4 (2-17) |
| Number of patients with previous EMR | 7 |
| **Histological diagnosis** | |
| NDBE | 99 (63%) |
| LGD | 21 (13.4%) |
| HGD | 24 (15.3%) |
| EC | 13 (8.3%) |

Association of biomarkers with dysplasia (per-biopsy analysis)

We first sought to identify a small biomarker panel that, when applied to tissue specimens, had the best diagnostic accuracy for prevalent dysplasia. To this end, we identified nine biomarkers that, based on the previous literature, had the most robust correlation with dysplasia in BE. [19-24] Figure 4 shows the strategy used for the generation of the panel. The 9 biomarkers were tested in the AFI-targeted biopsies from the original dataset to assess their association with dysplasia on a per-biopsy level. As shown in Table B,

**Table B.** Association of biomarkers with dysplasia

| Biomarker | Biomarker outcome | HGD/EC | | | Any dysplasia | | |
|---|---|---|---|---|---|---|---|
| | | No | Yes | P | No | Yes | P |
| HPP1 methylation | Negative | 59 (22.9%) | 3 (7.1%) | 0.02 | 56 (24.0%) | 6 (9.0%) | <0.01 |
| | Positive | 199 (77.1%) | 39 (92.9%) | | 177 (76.0%) | 61 (91.0%) | |
| RUNX3 methylation | Negative | 102 (39.5%) | 6 (14.3%) | <0.01 | 97 (41.6%) | 11 (16.4%) | <0.01 |
| | Positive | 156 (60.5%) | 36 (85.7%) | | 136 (58.4%) | 56 (83.6%) | |
| P16 methylation | Negative | 143 (55.4%) | 12 (28.6%) | <0.01 | 132 (56.7%) | 23 (34.3%) | <0.01 |
| | Positive | 115 (44.6%) | 30 (71.4%) | | 101 (43.3%) | 44 (65.7%) | |
| P53 IHC | Negative | 196 (70.5%) | 3 (6.8%) | <0.01 | 189 (76.8%) | 10 (13.2%) | <0.01 |
| | Positive | 82 (29.5%) | 41 (93.2%) | | 57 (23.2%) | 66 (86.8%) | |
| Cyclin A IHC | Negative | 238 (85.0%) | 8 (20.5%) | <0.01 | 223 (88.8%) | 23 (33.8%) | <0.01 |
| | Positive | 42 (15.0%) | 31 (79.5%) | | 28 (11.2%) | 45 (66.2%) | |
| Tetraploidy | Negative | 182 (69.2%) | 20 (57.1%) | 0.15 | 171 (72.2%) | 31 (50.8%) | <0.01 |
| | Positive | 81 (30.8%) | 15 (42.9%) | | 66 (27.8%) | 30 (48.2%) | |
| Aneuploidy | Negative | 232 (88.2%) | 10 (28.6%) | <0.01 | 216 (91.1%) | 26 (42.6%) | <0.01 |
| | Positive | 31 (11.8%) | 25 (71.4%) | | 21 (8.9%) | 35 (57.4%) | |
| 17p LOH | Negative | 112 (49.3%) | 7 (16.7%) | <0.01 | 107 (51.9%) | 12 (19.0%) | <0.01 |
| | Positive | 115 (50.7%) | 35 (83.3%) | | 99 (48.1 %) | 51 (81.0%) | |
| 9p LOH | Negative | 43 (19.2%) | 5 (12.2%) | 0.29 | 40 (19.5%) | 8 (13.3%) | 0.27 |
| | Positive | 181 (80.8%) | 36 (87.8%) | | 165 (80.5%) | 52 (86.7%) | |

all the biomarkers had a significantly higher percentage of positivity in biopsies taken from areas harboring definite dysplasia (LGD, HGD or EC) than from NDBE, with the exception of 9p LOH. When restricting the analysis to the association between biomarkers and HGD/EC, we found similar results, in that only tetraploidy and 9p LOH lacked statistical significance. Since large numbers of biomarkers are difficult to apply in the clinical setting, we used a strict statistical methodology to identify a small and clinically feasible biomarker panel to diagnose prevalent dysplasia. To this end, was used bootstrap resampling on the original database as well as on 5 imputed databases to account for missing values. We concentrated on the highest histological outcome for this analysis (HGD/EC), as this is what currently triggers therapeutic decisions according to clinical guidelines. We found that two IHC markers (p53 and cyclin A) and aneuploidy had the highest bootstrap inclusion frequency in all the 5 imputed databases, as well as in the original database (Figure 5A). This 3-biomarker had AUCs of 0.93 (95% CI: 0.89-0.98) and 0.97 (95%CI: 0.95-0.99) for a diagnosis of any grade of dysplasia and HGD/EC, respectively (Figure 5B-C). These data showed that these 3 biomarkers have the strongest association with dysplasia and could be combined into a panel to aid clinical diagnosis. Association between molecular biomarkers and AFI status

To overcome the limitation of non-uniform distribution of molecular changes in BE, we asked whether AFI positivity correlates with molecular abnormalities of the mucosa and therefore could be used to direct biopsies for biomarkers. To this end, we first analyzed the association between individual biomarker outcome and AFI status of the corresponding endoscopic area. Aneuploidy, 17p LOH, p53 IHC and cyclin A significantly associated with AFI positivity (p<0.05) (Supplementary Table 1).

**Supplementary Table 1.** Association of molecular biomarkers with AFI status

| Biomarker | Biomarker outcome | All areas | | | Non dysplastic areas | | |
|---|---|---|---|---|---|---|---|
| | | AFI- | AFI+ | P | AFI- | AFI+ | P |
| HPP1 methylation | Negative | 30 (23.8%) | 32 (18.3%) | 0.25 | 29 (25.2%) | 27 (22.9%) | 0.76 |
| | Positive | 96 (76.2%) | 143 (81.7%) | | 86 (74.8%) | 91 (77.1%) | |
| RUNX3 methylation | Negative | 50 (39.7%) | 58 (33.1%) | 0.27 | 49 (42.6%) | 48 (40.7%) | 0.79 |
| | Positive | 76 (60.3%) | 117 (66.9%) | | 66 (57.4%) | 70 (59.3%) | |
| P16 methylation | Negative | 71 (56.3%) | 84 (48.0%) | 0.16 | 67 (58.3%) | 65 (55.1%) | 0.69 |
| | Positive | 55 (43.7%) | 91 (52.0%) | | 48 (41.7%) | 53 (44.9%) | |
| P53 IHC | Negative | 96 (76.8%) | 103 (52.0%) | <0.01 | 93 (83.0%) | 96 (71.6%) | 0.05 |
| | Positive | 29 (23.2%) | 95 (48.0%) | | 19 (17.0%) | 38 (28.4%) | |
| Cyclin A IHC | Negative | 111 (84.1%) | 135 (72.2%) | 0.02 | 106 (89.1%) | 117 (88.6%) | 1.00 |
| | Positive | 21 (15.9%) | 52 (27.8%) | | 13 (10.9%) | 15 (11.4%) | |
| Tetraploidy | Negative | 89 (70.6%) | 114 (65.9%) | 0.45 | 85 (74.6%) | 86 (69.9%) | 0.47 |
| | Positive | 37 (29.4%) | 59 (34.1%) | | 29 (25.4%) | 37 (30.1%) | |
| Aneuploidy | Negative | 115 (91.3%) | 128 (74.0%) | <0.01 | 109 (95.6%) | 107 (87.0%) | 0.02 |
| | Positive | 11 (8.7%) | 45 (28%) | | 5 (4.4%) | 16 (13.0%) | |
| 17p LOH | Negative | 60 (53.6%) | 60 (38.0%) | 0.01 | 57 (55.9%) | 50 (48.1%) | 0.27 |
| | Positive | 52 (46.4%) | 98 (62.0%) | | 45 (44.1%) | 54 (51.9%) | |
| 9p LOH | Negative | 19 (17.0%) | 30 (19.5%) | 0.63 | 17 (16.7%) | 23 (22.3%) | 0.38 |
| | Positive | 93 (83.0%) | 124 (80.5%) | | 85 (83.3%) | 80 (77.7%) | |

Since dysplasia is enriched in AFI positive areas and could represent a confounding factor, we looked at this association after exclusion of areas harboring dysplasia and found that aneuploidy and p53 IHC retained significant association with AFI positivity (Supplementary Table 1). To confirm that this association between AFI positivity and biomarkers translate into a clinical advantage, we looked at whether the 3-biomarker panel (aneuploidy, p53 IHC and cyclin A) had a better accuracy for an overall diagnosis of HGD/EC if assessed on AFI+ areas than AFI- areas. We performed this comparative analysis in 5 imputed databases as well as in the original database (Figure 6). To avoid over-fitting, we used the bootstrap samples as a training set and patients not selected in bootstrap sample as a validation set. Notably, average AUCs in all 6 databases were significantly higher for the 3-biomarker panel assessed on AFI+ areas compared to AFI- areas. We therefore concluded that AFI positivity is associated with an enrichment of biomarkers and is a suitable tool to guide biopsies for analysis of biomarkers. Internal validation of the 3-biomaker panel (per-patient analysis)

In order to validate the panel of biomarkers selected in the per-biopsy analysis by the logistic regression model, we tested the accuracy for HGD/EC of different biomarker panels in our patient database. From a pool of 9 biomarkers, there are 84 different combinations of 3 biomarkers and 36 combinations of 2 biomarkers. We calculated the diagnostic accuracy of these 120 biomarker panels with different cut-offs of positive biomarkers in the 2000 bootstrap samples generated from the original database. The best diagnostic accuracy was achieved by the same panel identified in the per-biopsy analysis (p53 IHC, cyclin A IHC and aneuploidy), with a cut-off of 2 biomarkers positive to diagnose HGD/EC (Supplementary Table 2).

**Supplementary Table 2.** Twenty best panels of biomarkers for the diagnosis of HGD/EC from a total pool of 84 different combinations of 3 biomarkers and 36 combinations of 2 biomarkers with different cut-off values. Cut-off refers to the number of abnormal biomarkers required to yield a positive outcome. For each cut off the mean of the diagnostic accuracy form 2000 bootstrap samples and the standard deviation are represented. Highlighted in bold the best cut off for each panel.

| Biomarker 1 | Biomarker 2 | Biomarker 3 | Mean | | | Standard deviation | | |
|---|---|---|---|---|---|---|---|---|
| | | | cut-off 1 | cut-off 2 | cut-off 3 | cut-off 1 | cut-off 2 | cut-off 3 |
| Cyclin A IHC | p53 IHC | aneuploidy | 0.765272 | **0.866055** | 0.780642 | 0.02539 4 | 0.037145 | 0.048938 |
| Cyclin A IHC | p53 IHC | tetraploidy | 0.682272 | **0.859118** | 0.709647 | 0.024396 | 0.033878 | 0.051788 |
| Cyclin A IHC | p53 IHC | P53 LOH | 0.683017 | **0.856624** | 0.798002 | 0.024707 | 0.034406 | 0.047969 |
| Cyclin A IHC | p53 IHC | p16 methyl | 0.683315 | **0.839828** | 0.76563 | 0.024397 | 0.034727 | 0.04832 6 |
| p53 IHC | tetraploidy | P53 LOH | 0.635249 | **0.836269** | 0.65821 | 0.024351 | 0.036256 | 0.05332 6 |
| Cyclin A IHC | p53 IHC | N/A | 0.778035 | **0.83559** | N/A | 0.023653 | 0.040751 | N/A |
| Cyclin A IHC | tetraploidy | aneuploidy | 0.707605 | **0.835231** | 0.658476 | 0.030779 | 0.042051 | 0.047343 |
| Cyclin A IHC | p53 IHC | RUNX3 methyl | 0.632646 | 0.799535 | **0.83462 1** | 0.022021 | 0.035843 | 0.044334 |
| Cyclin A IHC | aneuploidy | RUNX3 methyl | 0.641786 | **0.83203 4** | 0.752845 | 0.023248 | 0.040472 | 0.050709 |
| Cyclin A IHC | p53 IHC | p16 LOH | 0.599525 | **0.827687** | 0.793979 | 0.020432 | 0.025124 | 0.047418 |
| Cyclin A IHC | aneuploidy | P53 LOH | 0.679156 | **0.826015** | 0.712769 | 0.038479 | 0.045307 | 0.050269 |
| p53 IHC | RUNX3 methyl | N/A | 0.632093 | **0.811182** | N/A | 0.028636 | 0.040024 | N/A |
| Cyclin A IHC | RUNX3 methyl | N/A | 0.646782 | **0.808857** | N/A | 0.028894 | 0.044359 | N/A |
| p53 IHC | HPP1 methyl | P53 LOH | 0.537662 | 0.706159 | **0.806901** | 0.013877 | 0.034944 | 0.046313 |
| p53 IHC | p53 LOH | N/A | 0.71064 | **0.801271** | N/A | 0.030795 | 0.045302 | N/A |
| Cyclin A IHC | p53 IHC | HPP1 methyl | 0.540834 | 0.815344 | **0.805547** | 0.013671 | 0.024707 | 0.046789 |
| Cyclin A IHC | p53 IHC | P53 LOH | 0.683017 | 0.856624 | **0.798002** | 0.024707 | 0.034406 | 0.047969 |
| p53 IHC | RUNX3 methyl | P53 LOH | 0.609351 | 0.778615 | **0.79585 8** | 0.021577 | 0.03136 | 0.046959 |
| Cyclin A IHC | p53 IHC | P53 LOH | 0.599525 | 0.827687 | **0.79397 9** | 0.020432 | 0.025124 | 0.047418 |
| p53 IHC | aneuploidy | N/A | 0.78885 | **0.79205** | N/A | 0.031008 | 0.049292 | N/A |

We therefore took this panel forward to predict histological outcome in the patient cohort with the 3-biomarker full dataset on AFI-targeted biopsies (n= 112) (Figure 4B). In keeping with the results from bootstrap resampling, a cut-off of 2 biomarkers positive had the best accuracy (Supplementary Table 3).

**Supplementary Table 3.** Detailed biomarker outcome in patients with a complete 3-biomaker panel dataset.

| Number of positive biomarkers | Number of patients with NDBE/LGD (%) | Number of patients with HGD/EC (%) | Total of patients | Sensitivity | Specificity |
|---|---|---|---|---|---|
| 0 | 50 (100.0%) | 0(0) | 50 | N/A | N/A |
| 1 | 28 (96.6%) | 1 (3.4%) | 29 | 100% | 56.8% |
| 2 | 8 (53.3%) | 7 (46.7%) | 15 | 95.8% | 88.6% |
| 3 | 2 (11.1%) | 16 (88.9%) | 18 | 66.7% | 97.7% |
| Total of patients | 88 | 24 | 112 | | |

Using this cut-off to identify patients with high histological grade, only one patient with HGD was mis-classified as having a low-risk biomarker signature (Figure 7 and Table C).

**Table C.** Comparison between 3-biomarker panel on AFI+ areas and gold standard. Seattle protocol includes all biopsies taken on HRE white light endoscopy (random + targeted on macroscopically visible abnormal areas). AFI + biomarkers column includes only the biopsies from AFI+ areas processed for the 3-biomaker panel. N/A: not applicable

| | Seattle protocol + histology | AFI + Biomarkers | p value |
|---|---|---|---|
| No. of HGD missed | 1 | 1 | N/A |
| Total no. of biopsies | 1385 | 310 | N/A |
| No of biopsies per patient | 12.4 | 2.8 | <0.001 |
| No of biopsies for every HGD/EC case diagnosed | 60.2 | 13.5 | N/A |

The biomarker panel had a sensitivity and a specificity of 95.8% (95%CI: 76.9%-99.8%) and 88.6% (95%CI: 79.7%-94.1%), respectively, for a diagnosis of HGD/EC, and 74.4% (95%CI: 57.6%-86.4%) and 94.5% (95%CI: 85.8%-98.2%), respectively, for diagnosis of any grade of dysplasia. This translated into a similar diagnostic accuracy to the Seattle protocol, which also missed one patient with HGD/EC (Table C). The gold standard had a sensitivity of 95.8% (95%CI: 76.9%-99.8%) for a diagnosis of HGD/EC (p=1.0 when compared with 3-biomarker panel) and 84.6% (95%CI: 68.8%-93.6%) for a diagnosis of any grade of dysplasia (p=0.26). Importantly, using this novel approach 2.8 biopsies per patient were taken on average compared with 12.8 for the standard biopsy protocol (p<0.001) (Table C). We concluded that this biomarker panel assessed on AFI-targeted biopsies has similar sensitivity for HGD/EC compared to the gold standard and allows a significant reduction in the number of biopsies required.

**Discussion of Example 2**

The current endoscopic surveillance strategy for detection of dysplasia and early cancer in patients with BE has several limitations including subjectivity of the histopathological diagnosis, sampling error, as well as high costs and risks related to large number of biopsies. In order to overcome these limitations researchers have focused on two conceptually independent areas, i.e., improvement of endoscopic imaging to detect inconspicuous dysplasia, and identification of molecular biomarkers to aid diagnosis of dysplasia. We devised a novel strategy in which advanced endoscopic imaging can be used to direct biopsy sampling for biomarkers and furthermore application of biomarkers may provide a more objective read-out than dysplasia. Single biomarkers or panels of biomarkers have been shown to correlate with dysplasia in BE [20, 22, 23, 32] and in some cases to estimate the individual risk of progression [22, 23, 32, 36, 37]. This prospective multi-centre phase-4 study evaluated nine biomarkers with the most robust published data. Our results confirmed that eight of them significantly associated with any grade of dysplasia and seven with HGD/EC. This is an important finding as to our knowledge this is the first study that has tested such a large panel of biomarkers in a single prospective patient cohort providing validation in an independent laboratory. The relevance of using panels instead of individual biomarkers resides in the evidence that a combination of biomarkers performs better than single biomarkers on their own, and this was confirmed previously in BE [32].

Applicability of biomarkers relies on the feasibility of laboratory assays in clinical practice, low costs and limited number of assays required if combined into a panel. A stringent statistical methodology, which took into account missing data and assessed the strength of different combinations of biomarkers, indicated the that p53 IHC, cyclin A IHC and aneuploidy constitute the panel with the best diagnostic accuracy for HGD and early cancer. This panel has the potential to satisfy criteria for clinical applicability. Two of the three biomarkers (p53 and cyclin A) rely on IHC, which is a routine technique in pathology laboratories. Of these, p53 is currently already employed by some pathologists to aid in the diagnosis of dysplasia. [19,20] For the evaluation of aneuploidy, we used a methodology based on FACS analysis from frozen samples preserved in DMSO, as this is the gold standard and we intended to validate previously published results. [32] For future clinical applications image cytometry (IC) is an alternative technique applicable to paraffin sections and has been previously proved to be comparable to flow cytometry in BE. [38]

There is evidence that molecular events and mutations occurring in BE are not distributed uniformly within the segment of columnar-lined esophagus. [24, 25] Hence, we postulated that the reliance on one or few random biopsies to assess biomarkers is subject to sampling error analogous to that seen for dysplasia. In recent years, a large effort has been invested in novel imaging modalities for the detection of inconspicuous neoplasia. AFI is promising, but its applicability in clinical practice has been limited by a high false positive rate. We hypothesized that AFI false positivity might at least in part depend on the field of a molecular abnormality surrounding or preceding subtle histological changes. Indeed, we found that aneuploidy and p53 abnormalities significantly correlated with AFI positivity independently of dysplasia and had bearing on the overall dysplasia status of the patient. DNA is known to be a weak fluorophore, therefore the loss of green autofluorescence may be explained by the increased DNA content observed in aneuploidy. Similarly, *p53* is a key gene in the control of DNA amplification and proliferation of aneuploid cells. [39] Even though the other biomarker in the panel (cyclin A) was not associated with AFI positivity in non-dysplastic areas, there is evidence that cyclin A can be overexpressed as result of cell cycle deregulation leading to aneuploidy. [40]

Notably, the new diagnostic tool of the invention proved to have the same sensitivity for dysplasia as the Seattle protocol with significantly fewer biopsies. This could be further reduced using IC to assess aneuploidy on the same diagnostic biopsy used for immunohistochemical markers. Therefore, this novel risk stratification approach, which is based on objectively measurable outcomes on a limited number of biopsies, has the potential to overcome several of the major limitations of BE surveillance endoscopy, including the sampling error and the subjectivity of a dysplasia diagnosis. In addition, the small number of biopsies may lead to shorter endoscopic procedures, lower risk of complications and improved cost-effectiveness. From an economic perspective, we estimated that the costs required to assess in our patient cohort the 3-biomaker panel (aneuploidy by IC + IHC for p53 and cyclin A) on biopsies targeted by AFI were similar to the costs of the Seattle protocol.

This is a cross-sectional study designed to assess the ability of biomarkers to diagnose prevalent dysplasia and therefore we did not set out to analyze follow up data and look at prediction of future cancer risk by biomarkers. However, it is notable that among the ten patients with a high risk biomarker profile (≥2 abnormal biomarkers) and no evidence of HGD/EC at the time of the endoscopy (Table 3), six of them (60%) had pathological progression within six months (four from LGD to HGD/EC, one from ID to EC and one from ID to LGD). Assuming this could have been prevalent disease missed by sampling error, this confirms that biomarkers overcome this limitation of the Seattle protocol.

This is the first study designed prospectively to assess a pre-defined group of multiple biomarkers in a large cohort of patients with BE. A single laboratory assessed a large panel of biomarkers that had previously been studied by independent groups in different patient cohorts. Finally the endoscopic protocol was carefully monitored to guarantee proper training and strict adherence.

In this example, only approximately 70% of participants could enter the per-patient analysis in the original database (112 out of 157). This is due to the fact that at the start of the study we lacked information about which of the 9 biomarkers would outperform the others in the statistical analysis. Therefore, since not all the AFI+ areas were large enough to allow for three research biopsies, we evenly distributed different biomarker analyses among the available biopsies and then used a combination of MI and bootstrap resampling to deal with missing data. [42]

In this example, assessment of dysplasia in the AFI-targeted area relied on one diagnostic biopsy only (processed for routine histopathological assessment) and the additional frozen biopsies were taken in close proximity to it and assumed to contain the same pathological grade. This was necessary since processing of freshly frozen biopsies for flow cytometry precluded histopathological assessment. Therefore there is a possibility of sampling error for very focal dysplasia. Although this may have led to an over-estimation of the biomarker enrichment in non-dysplastic areas, we believe that the high number of biopsies analyzed has minimized this potential bias.

In this example, this patient cohort was enriched for dysplasia, therefore it does not reflect the general BE population. However, enrichment for high risk patients was necessary to guarantee an adequate number of pathologic outcomes to correlate with biomarker positivity.

AFI is not compatible with all the endoscopic technologies available across the world. If appropriate, other advanced imaging modalities could be used to target biopsies for biomarker assessment.

In conclusion, this study demonstrates and provides evidence that a 3-biomarker panel on AFI-targeted biopsies has equivalent diagnostic accuracy for dysplasia compared to the gold standard and has numerous advantages including a significant reduction in the number of biopsies required and/or positive impact on the cost-effectiveness of endoscopic surveillance for BE.

**References to Example 2:**

1. Thrift AP, Whiteman DC. The incidence of esophageal adenocarcinoma continues to rise: analysis of period and birth cohort effects on recent trends. Ann Oncol 2012, Jul 30 [Epub ahead of print].

2. CancerResearchUK. http://www.cancerresearchuk.org/cancer-info/cancerstats/types/oesophagus/?script=true. Sept 2012.

3. Eloubeidi MA, Mason AC, Desmond RA, et al. Temporal trends (1973-1997) in survival of patients with esophageal adenocarcinoma in the United States: a glimmer of hope? Am J Gastroenterol 2003;98:1627-33.

4. Reid BJ, Li X, Galipeau PC, et al. Barrett's oesophagus and oesophageal adenocarcinoma: time for a new synthesis. Nat Rev Cancer 2010;10:87-101.

5. Cooper GS, Kou TD, Chak A. Receipt of previous diagnoses and endoscopy and outcome from esophageal adenocarcinoma: a population-based study with temporal trends. Am J Gastroenterol 2009;104:1356-62.

6. Spechler SJ, Sharma P, Souza RF, et al. American Gastroenterological Association technical review on the management of Barrett's esophagus. Gastroenterology 2011;140:e18-52.

7. Watson A, Heading RC, Shepherd NA, et al. Guidelines for the diagnosis and management of Barrett's columnar-lined oesophagus. http://www.bsg.org.uk/image/stories/docs/clinical/guidelines/oesophageal/Barretts_Oe s.pdf 2005.

8. Shaheen NJ, Sharma P, Overholt BF, et al. Radiofrequency ablation in Barrett's esophagus with dysplasia. N Engl J Med 2009;360:2277-88.

9. Abrams JA, Kapel RC, Lindberg GM, et al. Adherence to biopsy guidelines for Barrett's esophagus surveillance in the community setting in the United States. Clin Gastroenterol Hepatol 2009;7:736-42.

10. Peters FP, Curvers WL, Rosmolen WD, et al. Surveillance history of endoscopically treated patients with early Barrett's neoplasia: nonadherence to the Seattle biopsy protocol leads to sampling error. Dis Esophagus 2008;21:475-9.

11. Cameron AJ, Carpenter HA. Barrett's esophagus, high-grade dysplasia, and early adenocarcinoma: a pathological study. Am J Gastroenterol 1997;92:586-91.

12. Desai TK, Krishnan K, Samala N, et al. The incidence of oesophageal adenocarcinoma in non-dysplastic Barrett's oesophagus: a meta-analysis. Gut 2012;61:970-6.

13. Hvid-Jensen F, Pedersen L, Drewes AM, et al. Incidence of adenocarcinoma among patients with Barrett's esophagus. N Engl J Med 2011;365:1375-83.

14. Curvers WL, Herrero LA, Wallace MB, et al. Endoscopic tri-modal imaging is more effective than standard endoscopy in identifying early-stage neoplasia in Barrett's esophagus. Gastroenterology 2010;139:1106-14.

15. Curvers WL, van Vilsteren FG, Baak LC, et al. Endoscopic trimodal imaging versus standard video endoscopy for detection of early Barrett's neoplasia: a multicenter, randomized, crossover study in general practice. Gastrointest Endosc 2011;73:195-203.

16. Kara M, DaCosta RS, Wilson BC, et al. Autofluorescence-based detection of early neoplasia in patients with Barrett's esophagus. Dig Dis 2004;22:134-41.

17. Curvers WL, ten Kate FJ, Krishnadath KK, et al. Low-grade dysplasia in Barrett's esophagus: overdiagnosed and underestimated. Am J Gastroenterol 2010;105:1523-30.

18. Wani S, Falk GW, Post J, et al. Risk factors for progression of low-grade dysplasia in patients with Barrett's esophagus. Gastroenterology 2011;141:1179-86, 1186 e1.

19. Jouret-Mourin A, Sempoux C, Due KH, et al. Usefulness of histopathological markers in diagnosing Barrett's intraepithelial neoplasia (dysplasia). Acta Gastroenterol Belg 2009;72:425-32.

20. Kaye PV, Haider SA, Ilyas M, et al. Barrett's dysplasia and the Vienna classification: reproducibility, prediction of progression and impact of consensus reporting and p53 immunohistochemistry. Histopathology 2009;54:699-712.

21. Skacel M, Petras RE, Gramlich TL, et al. The diagnosis of low-grade dysplasia in Barrett's esophagus and its implications for disease progression. Am J Gastroenterol 2000;95:3383-7.

22. Lao-Sirieix P, Lovat L, Fitzgerald RC. Cyclin A immunocytology as a risk stratification tool for Barrett's esophagus surveillance. Clin Cancer Res 2007;13:659-65.

23. Schulmann K, Sterian A, Berki A, et al. Inactivation of p16, RUNX3, and HPP1 occurs early in Barrett's-associated neoplastic progression and predicts progression risk. Oncogene 2005;24:4138-48.

24. Galipeau PC, Prevo LJ, Sanchez CA, et al. Clonal expansion and loss of heterozygosity at chromosomes 9p and 17p in premalignant esophageal (Barrett's) tissue. J Natl Cancer Inst 1999;91:2087-95.

25. Leedham SJ, Preston SL, McDonald SA, et al. Individual crypt genetic heterogeneity and the origin of metaplastic glandular epithelium in human Barrett's oesophagus. Gut 2008;57:1041-8.

26. Boerwinkel DF, Di Pietro M, Liu X, et al. Endoscopic TriModal imaging and biomarkers for neoplasia conjoined: a feasibility study in Barrett's esophagus. Dis Esophagus 2012;Oct 15, Epub ahead of print.

27. Curvers WL, Singh R, Wallace MB, et al. Identification of predictive factors for early neoplasia in Barrett's esophagus after autofluorescence imaging: a stepwise multicenter structured assessment. Gastrointest Endosc 2009;70:9-17.

28. Dixon MF. Gastrointestinal epithelial neoplasia: Vienna revisited. Gut 2002;51:130-1.

29. Bennett C, Vakil N, Bergman J, et al. Consensus Statements for Management of Barrett's Dysplasia and Early-Stage Esophageal Adenocarcinoma, Based on a Delphi Process. Gastroenterology 2012;143:336-46.

30. Reid BJ, Haggitt RC, Rubin CE, et al. Barrett's esophagus. Correlation between flow cytometry and histology in detection of patients at risk for adenocarcinoma. Gastroenterology 1987;93:1-11.

31. Depledge DP, Evans KJ, Ivens AC, et al. Comparative expression profiling of Leishmania: modulation in gene expression between species and in different host genetic backgrounds. PLoS Negl Trop Dis 2009;3:e476.

32. Galipeau PC, Li X, Blount PL, et al. NSAIDs modulate CDKN2A, TP53, and DNA content risk for progression to esophageal adenocarcinoma. PLoS Med 2007;4:e67.

33. Su YS, Gelman A, Hill J, et al. Multiple imputation with diagnostics (mi) in R: Opening windows into the black box. Journal of Statistical Software 2011; 10.

34. Sauerbrei W. The use of resampling methods to simplify regression models in medical statistics. Appl. Statist. 1999;48:313-329.

35. Sauerbrei W. The use of resampling methods to simplify regression models in medical statistics. Journal of the Royal Statistical Society: Series C (Applied Statistics) 1998;48:313-329.

36. Murray L, Sedo A, Scott M, et al. TP53 and progression from Barrett's metaplasia to oesophageal adenocarcinoma in a UK population cohort. Gut 2006;55:1390-7.

37. Kastelein F, Biermann K, Steyerberg EW, et al. Aberrant p53 protein expression is associated with an increased risk of neoplastic progression in patients with Barrett's oesophagus. Gut 2012;Dec 20, Epub ahead of print.

38. Dunn JM, Mackenzie GD, Oukrif D, et al. Image cytometry accurately detects DNA ploidy abnormalities and predicts late relapse to high-grade dysplasia and adenocarcinoma in Barrett's oesophagus following photodynamic therapy. Br J Cancer 2010;102:1608-17.

39. Fukasawa K, Choi T, Kuriyama R, et al. Abnormal centrosome amplification in the absence of p53. Science 1996;271:1744-7.

40. Gardner L, Malik R, Shimizu Y, et al. Geminin overexpression prevents the completion of topoisomerase IIalpha chromosome decatenation, leading to aneuploidy in human mammary epithelial cells. Breast Cancer Res 2011;13:R53.

41. di Pietro M, Fitzgerald RC. Screening and risk stratification for Barrett's esophagus: how to limit the clinical impact of the increasing incidence of esophageal adenocarcinoma. Gastroenterol Clin North Am 2013;42:155-73.

42. Heymans MW, van Buuren S, Knol DL, et al. Variable selection under multiple imputation using the bootstrap in a prognostic study. BMC Med Res Methodol 2007;7:33.

**References to main text**

1. Pohl, H., B. Sirovich, and H.G. Welch, Esophageal adenocarcinoma incidence: are we reaching the peak? Cancer Epidemiol Biomarkers Prev, 2010. 19(6): p. 1468-70.

2. Eloubeidi, M.A., et al., Temporal trends (1973-1997) in survival of patients with esophageal adenocarcinoma in the United States: a glimmer of hope? Am J Gastroenterol, 2003. 98(7): p. 1627-33.

3. Reid, B.J., et al., Barrett's oesophagus and oesophageal adenocarcinoma: time fora new synthesis. Nat Rev Cancer, 2010. 10(2): p. 87-101.

4. Spechler, S.J., et al., American Gastroenterological Association technical review on the management of Barrett's esophagus. Gastroenterology, 2011. 140(3): p. e18-52; quiz e13.

5. Wang, K.K. and R.E. Sampliner, Updated guidelines 2008 for the diagnosis, surveillance and therapy of Barrett's esophagus. Am J Gastroenterol, 2008. 103(3): p. 788-97.

6. Hirota, W.K., et al., ASGE guideline: the role of endoscopy in the surveillance of premalignant conditions of the upper GI tract. Gastrointest Endosc, 2006. 63(4): p. 570-80.

7. Armstrong, D., et al., Canadian Consensus Conference on the management of gastroesophageal reflux disease in adults - update 2004. Can J Gastroenterol, 2005. 19(1): p. 15-35.

8. Watson A, H.R.S.N., British Society of Gastroenterology, Guidelines for the diagnosis and management of Barrett's columnar-lined oesophagus. Available at: http://www.bsg.org.uk, 2005.

9. Shaheen, N.J., et al., Radiofrequency ablation in Barrett's esophagus with dysplasia. N Engl J Med, 2009. 360(22): p. 2277-88.

10. Wani, S., et al., Esophageal adenocarcinoma in Barrett's esophagus after endoscopic ablative therapy: a meta-analysis and systematic review. Am J Gastroenterol, 2009. 104(2): p. 502-13.

11. Curvers, W.L., et al., Endoscopic tri-modal imaging for detection of early neoplasia in Barrett's oesophagus: a multi-centre feasibility study using high-resolution endoscopy, autofluorescence imaging and narrow band imaging incorporated in one endoscopy system. Gut, 2008. 57(2): p. 167-72.

12. Kerkhof, M., et al., Grading of dysplasia in Barrett's oesophagus: substantial interobserver variation between general and gastrointestinal pathologists. Histopathology, 2007. 50(7): p. 920-7.

13. Montgomery, E., et al., Reproducibility of the diagnosis of dysplasia in Barrett esophagus: a reaffirmation. Hum Pathol, 2001. 32(4): p. 368-78.

14. Wani, S., et al., Greater interobserver agreement by endoscopic mucosal resection than biopsy samples in Barrett's dysplasia. Clin Gastroenterol Hepatol, 2010. 8(9): p. 783-8.

15. Kahrilas, P.J., The problems with surveillance of Barrett's esophagus. N Engl J Med, 2011. 365(15): p. 1437-8.

16. Shaheen, N.J., et al., Is there publication bias in the reporting of cancer risk in Barrett's esophagus? Gastroenterology, 2000. 119(2): p. 333-8.

17. Bhat, S., et al., Risk of malignant progression in Barrett's esophagus patients: results from a large population-based study. J Natl Cancer Inst, 2011. 103(13): p. 1049-57.

18. Hvid-Jensen, F., et al., Incidence of adenocarcinoma among patients with Barrett's esophagus. N Engl J Med, 2011. 365(15): p. 1375-83.

19. Curvers, W.L., R. Kiesslich, and J.J. Bergman, Novel imaging modalities in the detection of oesophageal neoplasia. Best Pract Res Clin Gastroenterol, 2008. 22(4): p. 687-720.

20. Wolfsen, H.C., et al., Prospective, controlled tandem endoscopy study of narrow band imaging for dysplasia detection in Barrett's Esophagus. Gastroenterology, 2008. 135(1): p. 24-31.

21. Sharma, P., et al., Standard endoscopy with random biopsies versus narrow band imaging targeted biopsies in Barrett's oesophagus: a prospective, international, randomised controlled trial. Gut, 2012.

22. Kara, M., et al., Autofluorescence-based detection of early neoplasia in patients with Barrett's esophagus. Dig Dis, 2004. 22(2): p. 134-41.

23. Kara, M.A., et al., Endoscopic video autofluorescence imaging may improve the detection of early neoplasia in patients with Barrett's esophagus. Gastrointest Endosc, 2005. 61 (6): p. 679-85.

24. Curvers, W.L., et al., Endoscopic tri-modal imaging is more effective than standard endoscopy in identifying early-stage neoplasia in Barrett's esophagus. Gastroenterology, 2010. 139(4): p. 1106-14.

25. Curvers, W.L., et al., Endoscopic trimodal imaging versus standard video endoscopy for detection of early Barrett's neoplasia: a multicenter, randomized, crossover study in general practice. Gastrointest Endosc, 2011. 73(2): p. 195-203.

26. Kaye, P.V., et al., Barrett's dysplasia and the Vienna classification: reproducibility, prediction of progression and impact of consensus reporting and p53 immunohistochemistry. Histopathology, 2009. 54(6): p. 699-712.

27. Skacel, M., et al., p53 expression in low grade dysplasia in Barrett's esophagus: correlation with interobserver agreement and disease progression. Am J Gastroenterol, 2002. 97(10): p. 2508-13.

28. Murray, L, et al., TP53 and progression fro m Barrett's metaplasia to oesophageal adenocarcinoma in a UKpopulation cohort. Gut, 2006. 55(10): p. 1390-7.

29. Sikkema, M., et al., Aneuploidy and overexpression of Ki67 and p53 as markers for neoplastic progression in Barrett's esophagus: a case-control study. Am J Gastroenterol, 2009. 104(11): p. 2673-80.

30. Schulmann, K, et al., Inactivation of p16, RUNX3, and HPP1 occurs early in Barrett's-associated neoplastic progression and predicts progression risk. Oncogene, 2005. 24(25): p. 4138-48.

31. Galipeau, P.C., et al., NSAIDs mo dulate CDKN2A, TP53, and DNA c ontent risk for progression to e so p ha geal adenoc arcinoma. PLo SMed, 2007. 4(2): p. e67.

32. Lao-Sirieix, P., L Lovat, and RC. Fitzgerald, Cyclin A immunocytology as a risk stra tific a tio n tool for Barrett's esophagus surveillance. Clin Cancer Res, 2007. 13(2 Pt 1): p. 659-65.

33. Galipeau, P.C., et al., Clonal expansion and loss of heterozygosity at chromosomes 9p and 17p in premalignant esophageal (Barrett's) tissue. J Natl Cancer Inst, 1999. 91(24): p. 2087-95.

34. Leedham, S.J., et al., Individual crypt genetic heterogeneity and the origin of metaplastic glandular epithelium in human Barrett's oesophagus. Gut, 2008. 57(8): p. 1041-8.

35. Fukasawa, K, et al., Abnormalcentrosome amplification in the absence of p53. Science, 1996. 271(5256): p.

**EP 2 850 208 B1**

1744-7.

36. Thompson, S.L and D.A. Compton, Proliferation of aneuploid human cells is limited by a p53-dependentmechanism. J Cell Biol, 2010. 188(3): p. 369-81.

**Claims**

1. A method for determining if a subject has an increased like lihood of high grade dysplasia (HGD) oresophagaladeno carcinoma (EC), the method comprising;

   a) assaying a sample provided from an AFI positive patch of said subject's oesophagus for

   (i) aneuploidy, and
   (ii) abnormal p53, wherein assaying for p53 comprises immune histochemical staining of the sample for p53, and scoring the stain as normal or abnormal wherein either a strong, dark stain compared to background stain or an absence of stain compared to background stain is an abnormal stain; and
   (iii) abnormal Cyclin A, wherein Cyclin A is assayed by immune histochemical staining of the sample for Cyclin A, determining the percentage of Cyclin A positive epithelial surface cells compared to Cyclin A negative epithelial surface cells, wherein a percentage of 1% or more Cyclin A positive epithelial surface cells is an abnormal stain;

   b) wherein if said sample shows the presence of at least two of (i), (ii) and (iii), then said subject is determined as having an increased like lihood of high grade dysplasia (HGD) oresophagaladeno carcinoma (EC).

2. A method according to claim 1, wherein said sample is further assayed for one or more of 9p loss of heterozygosity (LOH), Runx3 methylation, p16 m e thyla tio n, 17p loss of heterozygosity (IDH), HPP1 methylation or G2 ploidy, wherein if one of more of said further markers are found in said sample, said subject is determined ashaving a further increased like lihood of dysplasia.

3. A method according to claim 1, wherein said sample is further assayed for one of more of 9p loss of heterozygosity (LOH), Runx3 hypermethylation, p16 hypermethylation, 17p loss of heterozygosity (LOH), HPP1 hypermethylation or G2 tetraploidy, wherein if one or more of said further markers are found in said sample, said subject is determined as having a further increased like lihood of dysplasia.

4. A method according to any of claims 1 to 3 wherein said sample is a biopsy.

5. A method according to any preceding claim wherein said sample is an endoscopic sample.

6. Method according to any preceding claim wherein aneuploidy is assayed by determining the DNA content by flow cytometry DNA analysis and inferring from said determination whether said DNA content is aneuploid.

**Patentansprüche**

1. Verfahren zum Bestimmen, ob ein Individuum eine erhöhte Wahrscheinlichkeit von hochgradiger Dysplasie (HGD) oder Ösophagus-Adenokarzinom (EC) aufweist, wobei das Verfahren Folgendes umfasst:

   a) Untersuchen einer Probe aus einem AFI-positiven Patch vom Ösophagus des Individuums auf

   (i) eine Euplodie und
   (ii) abnormes p53, wobei das Untersuchen auf p53 das immunhistochemische Färben der Probe auf p53 und das Bewerten der Färbung als normal oder abnormal umfasst, wobei entweder eine starke, dunkle Färbung im Vergleich mit der Hintergrundfärbung oder eine Abwesenheit von Färbung im Vergleich mit der Hintergrundfärbung eine abnormale Färbung ist, und
   (iii) abnormes Cyclin A, wobei Cyclin A durch das immunhistochemische Färben der Probe auf Cyclin A und Bestimmen des Prozentsatzes von Cyclin A-positiven epithelialen Oberflächenzellen im Vergleich mit Cyclin A-negativen epithelialen Oberflächenzellen untersucht wird, wobei ein Prozentsatz von 1 % oder mehr von Cyclin A-positiven epithelialen Oberflächenzellen eine abnormale Färbung ist,

b) wobei, falls die Probe die Anwesenheit von mindestens zwei von (i), (ii) und (iii) anzeigt, dann festgelegt wird, dass das Individuum eine erhöhte Wahrscheinlichkeit von hochgradiger Dysplasie (HGD) oder Ösophagus-Adenokarzinom (EC) aufweist.

2. Verfahren nach Anspruch 1, wobei die Probe ferner auf eines oder mehrere der Folgenden untersucht wird: 9p-Verlust von Heterozygotie (LOH), Runx3-Methylierung, p16-Methylierung, 17p-Verlust von Heterozygotie (LOH), HPP1-Methylierung oder G2-Ploidie, wobei, falls einer oder mehrere dieser weiteren Marker in der Probe gefunden werden, festgelegt wird, dass das Individuum eine weiter erhöhte Wahrscheinlichkeit von Dysplasie aufweist.

3. Verfahren nach Anspruch 1, wobei die Probe ferner auf eines oder mehrere der Folgenden untersucht wird: 9p-Verlust von Heterozygotie (LOH), Runx3-Hypermethylierung, p16-Hypermethylierung, 17p-Verlust von Heterozygotie (LOH), HPP1-Hypermethylierung oder G2-Tetraploidie, wobei, falls einer oder mehrere dieser weiteren Marker in der Probe gefunden werden, festgelegt wird, dass das Individuum eine weiter erhöhte Wahrscheinlichkeit von Dysplasie aufweist.

4. Verfahren nach einem der Ansprüche 1 bis 3, wobei die Probe eine Biopsie ist.

5. Verfahren nach einem vorhergehenden Anspruch, wobei die Probe eine endoskopische Probe ist.

6. Verfahren nach einem vorhergehenden Anspruch, wobei eine Euploidie durch Bestimmen des DNA-Gehalts durch Flusszytometrie-DNA-Analyse und Rückschließen aus dieser Bestimmung, ob der DNA-Gehalt euploid ist, untersucht wird.

**Revendications**

1. Procédé pour déterminer si un sujet a une probabilité accrue de dysplasie de haut degré (HGD) ou d'adénocarcinome de l'oesophage (EC), le procédé comprenant :

    a) le dosage d'un échantillon tiré d'un patch positif AFI de l'oesophage dudit sujet pour

       (i) une aneuploïdie, et
       (ii) une p53 anormale, dans lequel le dosage pour la p53 comprend une coloration immunohistochimique de l'échantillon pour la p53 et pour la cotation de la tache en tant que normale ou anormale, dans lequel une forte tache sombre en comparaison d'une tache de fond ou une absence de tache en comparaison d'une tache de fond est une tache anormale ; et
       (iii) une cycline A anormale, dans lequel la cycline A est dosée par coloration immunohistochimique de l'échantillon pour la cycline A, en déterminant le pourcentage de cellules de surface épithéliales positives de cycline A en comparaison de cellules de surface épithéliales négatives de cycline A, dans lequel un pourcentage de 1 % ou plus de cellules de surface épithéliales positives de cycline A est une tache anormale ;

    b) dans lequel, si ledit échantillon montre la présence d'au moins deux de (i), (ii) et (iii), ledit sujet est déterminé comme ayant une probabilité accrue de dysplasie de haut degré (HGD) ou d'adénocarcinome de l'oesophage (EC).

2. Procédé selon la revendication 1, dans lequel ledit échantillon est en outre dosé pour une ou plus de perte d'hétérozygotie (LOH) de 9p, de méthylation de Runx3, de méthylation de p16, de perte d'hétérozygotie (LOH) de 17p, de méthylation de HPP1 ou de ploïdie de G2, dans lequel, si un ou plusieurs desdits autres marqueurs est ou sont trouvés dans ledit échantillon, ledit sujet est déterminé comme ayant une plus grande probabilité de dysplasie.

3. Procédé selon la revendication 1, dans lequel ledit échantillon est en outre dosé pour une ou plus de perte d'hétérozygotie (LOH) de 9p, d'hyperméthylation de Runx3, d'hyperméthylation de p16, de perte d'hétérozygotie (LOH) de 17p, d'hyperméthylation de HPP1 ou de tétraploïdie de G2, dans lequel, si un ou plusieurs desdits autres marqueurs est ou sont trouvés dans ledit échantillon, ledit sujet est déterminé comme ayant une plus grande probabilité de dysplasie.

4. Procédé selon l'une quelconque des revendications 1 à 3, dans lequel ledit échantillon est une biopsie.

**5.** Procédé selon l'une quelconque des revendications précédentes, dans lequel ledit échantillon est un échantillon endoscopique.

**6.** Procédé selon l'une quelconque des revendications précédentes, dans lequel l'aneuploïdie est dosée en déterminant la teneur en ADN par analyse d'ADN par cytométrie en flux et en déduisant de ladite détermination si ladite teneur en ADN est aneuploïde.

## Figure 1

- 175 patients recruited
  - 16 patients excluded
    - ● Endoscopic criteria not met
    - ● Breech of bx protocol
- 158 patients completed protocol
    - ● 232 AFI+ targeted bx
    - ● 145 AFI- targeted bx
    - ● Per - biopsy analysis
    - ● Generation of a panel
  - 47 patients excluded
    - ● Lack of full biomarker panel set
- 111 patients with complete data
    - ● Per - patient analysis
    - ● Diagnostic accuracy

Figure 2

## Accuracy of the 3 biomarker panel

3 - biomarker panel:
p53 IHC, cyclin A IHC and aneuploidy

### Any dysplasia

AUC = 0.92 (0.87 - 0.98)

### HGD/EC

AUC = 0.95 (0.89 - 1.00)

# Figure 3

### AFI+ areas vs AFI- areas

## Patients with complete panel on AFI+ and AFI-

### Panel on AFI+ areas

AUC = 0.95 (0.90 - 1.00)

### Panel on AFI- areas

AUC = 0.75 (0.61 - 0.89)

# Figure 4

Figure 5

Figure 6

Figure 7

Figure 8

A

B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011133935 A

**Non-patent literature cited in the description**

- **KAYE PV ; HAIDER SA ; ILYAS M et al.** *Histopathology,* 2009, vol. 54, 699-712
- **GALIPEAU PC ; PREVO LJ ; SANCHEZ CA et al.** *J Natl Cancer Inst,* 1999, vol. 91, 2087-95
- **SCHULMANN, K et al.** *Oncogene,* 2005, vol. 24 (25), 4138-48
- **LAO-SIRIEIX, P. ; L LOVAT ; R.C. FITZGERALD.** *Clin Cancer Res,* 2007, vol. 13 (2), 659-65
- **THRIFT AP ; WHITEMAN DC.** The incidence of esophageal adenocarcinoma continues to rise: analysis of period and birth cohort effects on recent trends. *Ann Oncol,* 30 July 2012
- **ELOUBEIDI MA ; MASON AC ; DESMOND RA et al.** Temporal trends (1973-1997) in survival of patients with esophageal adenocarcinoma in the United States: a glimmer of hope?. *Am J Gastroenterol,* 2003, vol. 98, 1627-33
- **REID BJ ; LI X ; GALIPEAU PC et al.** Barrett's oesophagus and oesophageal adenocarcinoma: time for a new synthesis. *Nat Rev Cancer,* 2010, vol. 10, 87-101
- **COOPER GS ; KOU TD ; CHAK A.** Receipt of previous diagnoses and endoscopy and outcome from esophageal adenocarcinoma: a population-based study with temporal trends. *Am J Gastroenterol,* 2009, vol. 104, 1356-62
- **SPECHLER SJ ; SHARMA P ; SOUZA RF et al.** American Gastroenterological Association technical review on the management of Barrett's esophagus. *Gastroenterology,* 2011, vol. 140, e18-52
- **WATSON A ; HEADING RC ; SHEPHERD NA et al.** *Guidelines for the diagnosis and management of Barrett's columnar-lined oesophagus,* 2005, http://www.bsg.org.uk/image/stories/docs/clinical/guidelines/oesophageal/Barretts_Oe s.pdf
- **SHAHEEN NJ ; SHARMA P ; OVERHOLT BF et al.** Radiofrequency ablation in Barrett's esophagus with dysplasia. *N Engl J Med,* 2009, vol. 360, 2277-88
- **ABRAMS JA ; KAPEL RC ; LINDBERG GM et al.** Adherence to biopsy guidelines for Barrett's esophagus surveillance in the community setting in the United States. *Clin Gastroenterol Hepatol,* 2009, vol. 7, 736-42
- **PETERS FP ; CURVERS WL ; ROSMOLEN WD et al.** Surveillance history of endoscopically treated patients with early Barrett's neoplasia: nonadherence to the Seattle biopsy protocol leads to sampling error. *Dis Esophagus,* 2008, vol. 21, 475-9
- **CAMERON AJ ; CARPENTER HA.** Barrett's esophagus, high-grade dysplasia, and early adenocarcinoma: a pathological study. *Am J Gastroenterol,* 1997, vol. 92, 586-91
- **DESAI TK ; KRISHNAN K ; SAMALA N et al.** The incidence of oesophageal adenocarcinoma in non-dysplastic Barrett's oesophagus: a meta-analysis. *Gut,* 2012, vol. 61, 970-6
- **HVID-JENSEN F ; PEDERSEN L ; DREWES AM et al.** Incidence of adenocarcinoma among patients with Barrett's esophagus. *N Engl J Med,* 2011, vol. 365, 1375-83
- **CURVERS WL ; HERRERO LA ; WALLACE MB et al.** Endoscopic tri-modal imaging is more effective than standard endoscopy in identifying early-stage neoplasia in Barrett's esophagus. *Gastroenterology,* 2010, vol. 139, 1106-14
- **CURVERS WL ; VAN VILSTEREN FG ; BAAK LC et al.** Endoscopic trimodal imaging versus standard video endoscopy for detection of early Barrett's neoplasia: a multicenter, randomized, crossover study in general practice. *Gastrointest Endosc,* 2011, vol. 73, 195-203
- **KARA M ; DACOSTA RS ; WILSON BC et al.** Autofluorescence-based detection of early neoplasia in patients with Barrett's esophagus. *Dig Dis,* 2004, vol. 22, 134-41
- **CURVERS WL ; TEN KATE FJ ; KRISHNADATH KK et al.** Low-grade dysplasia in Barrett's esophagus: overdiagnosed and underestimated. *Am J Gastroenterol,* 2010, vol. 105, 1523-30

- **WANI S ; FALK GW ; POST J et al.** Risk factors for progression of low-grade dysplasia in patients with Barrett's esophagus. *Gastroenterology,* 2011, vol. 141 (1179-86), 1186
- **JOURET-MOURIN A ; SEMPOUX C ; DUE KH et al.** Usefulness of histopathological markers in diagnosing Barrett's intraepithelial neoplasia (dysplasia). *Acta Gastroenterol Belg,* 2009, vol. 72, 425-32
- **KAYE PV ; HAIDER SA ; ILYAS M et al.** Barrett's dysplasia and the Vienna classification: reproducibility, prediction of progression and impact of consensus reporting and p53 immunohistochemistry. *Histopathology,* 2009, vol. 54, 699-712
- **SKACEL M ; PETRAS RE ; GRAMLICH TL et al.** The diagnosis of low-grade dysplasia in Barrett's esophagus and its implications for disease progression. *Am J Gastroenterol,* 2000, vol. 95, 3383-7
- **LAO-SIRIEIX P ; LOVAT L ; FITZGERALD RC.** Cyclin A immunocytology as a risk stratification tool for Barrett's esophagus surveillance. *Clin Cancer Res,* 2007, vol. 13, 659-65
- **SCHULMANN K ; STERIAN A ; BERKI A et al.** Inactivation of p16, RUNX3, and HPP1 occurs early in Barrett's-associated neoplastic progression and predicts progression risk. *Oncogene,* 2005, vol. 24, 4138-48
- **GALIPEAU PC ; PREVO LJ ; SANCHEZ CA et al.** Clonal expansion and loss of heterozygosity at chromosomes 9p and 17p in premalignant esophageal (Barrett's) tissue. *J Natl Cancer Inst,* 1999, vol. 91, 2087-95
- **LEEDHAM SJ ; PRESTON SL ; MCDONALD SA et al.** Individual crypt genetic heterogeneity and the origin of metaplastic glandular epithelium in human Barrett's oesophagus. *Gut,* 2008, vol. 57, 1041-8
- **BOERWINKEL DF ; DI PIETRO M ; LIU X et al.** Endoscopic TriModal imaging and biomarkers for neoplasia conjoined: a feasibility study in Barrett's esophagus. *Dis Esophagus,* 15 October 2012
- **CURVERS WL ; SINGH R ; WALLACE MB et al.** Identification of predictive factors for early neoplasia in Barrett's esophagus after autofluorescence imaging: a stepwise multicenter structured assessment. *Gastrointest Endosc,* 2009, vol. 70, 9-17
- **DIXON MF.** Gastrointestinal epithelial neoplasia: Vienna revisited. *Gut,* 2002, vol. 51, 130-1
- **BENNETT C ; VAKIL N ; BERGMAN J et al.** Consensus Statements for Management of Barrett's Dysplasia and Early-Stage Esophageal Adenocarcinoma, Based on a Delphi Process. *Gastroenterology,* 2012, vol. 143, 336-46
- **REID BJ ; HAGGITT RC ; RUBIN CE et al.** Barrett's esophagus. Correlation between flow cytometry and histology in detection of patients at risk for adenocarcinoma. *Gastroenterology,* 1987, vol. 93, 1-11
- **DEPLEDGE DP ; EVANS KJ ; IVENS AC et al.** Comparative expression profiling of Leishmania: modulation in gene expression between species and in different host genetic backgrounds. *PLoS Negl Trop Dis,* 2009, vol. 3, e476
- **GALIPEAU PC ; LI X ; BLOUNT PL et al.** NSAIDs modulate CDKN2A, TP53, and DNA content risk for progression to esophageal adenocarcinoma. *PLoS Med,* 2007, vol. 4, e67
- **SU YS ; GELMAN A ; HILL J et al.** Multiple imputation with diagnostics (mi) in R: Opening windows into the black box. *Journal of Statistical Software,* 2011, vol. 10
- **SAUERBREI W.** The use of resampling methods to simplify regression models in medical statistics. *Appl. Statist.,* 1999, vol. 48, 313-329
- **SAUERBREI W.** The use of resampling methods to simplify regression models in medical statistics. *Journal of the Royal Statistical Society: Series C (Applied Statistics),* 1998, vol. 48, 313-329
- **MURRAY L ; SEDO A ; SCOTT M et al.** TP53 and progression from Barrett's metaplasia to oesophageal adenocarcinoma in a UK population cohort. *Gut,* 2006, vol. 55, 1390-7
- **KASTELEIN F ; BIERMANN K ; STEYERBERG EW et al.** Aberrant p53 protein expression is associated with an increased risk of neoplastic progression in patients with Barrett's oesophagus. *Gut,* 20 December 2012
- **DUNN JM ; MACKENZIE GD ; OUKRIF D et al.** Image cytometry accurately detects DNA ploidy abnormalities and predicts late relapse to high-grade dysplasia and adenocarcinoma in Barrett's oesophagus following photodynamic therapy. *Br J Cancer,* 2010, vol. 102, 1608-17
- **FUKASAWA K ; CHOI T ; KURIYAMA R et al.** Abnormal centrosome amplification in the absence of p53. *Science,* 1996, vol. 271, 1744-7
- **GARDNER L ; MALIK R ; SHIMIZU Y et al.** Geminin overexpression prevents the completion of topoisomerase IIalpha chromosome decatenation, leading to aneuploidy in human mammary epithelial cells. *Breast Cancer Res,* 2011, vol. 13, R53
- **DI PIETRO M ; FITZGERALD RC.** Screening and risk stratification for Barrett's esophagus: how to limit the clinical impact of the increasing incidence of esophageal adenocarcinoma. *Gastroenterol Clin North Am,* 2013, vol. 42, 155-73
- **HEYMANS MW ; VAN BUUREN S ; KNOL DL et al.** Variable selection under multiple imputation using the bootstrap in a prognostic study. *BMC Med Res Methodol,* 2007, vol. 7, 33
- **POHL, H. ; B. SIROVICH ; H.G. WELCH.** Esophageal adenocarcinoma incidence: are we reaching the peak?. *Cancer Epidemiol Biomarkers Prev,* 2010, vol. 19 (6), 1468-70

- **ELOUBEIDI, M.A. et al.** Temporal trends (1973-1997) in survival of patients with esophageal adenocarcinoma in the United States: a glimmer of hope?. *Am J Gastroenterol,* 2003, vol. 98 (7), 1627-33
- **REID, B.J. et al.** Barrett's oesophagus and oesophageal adenocarcinoma: time fora new synthesis. *Nat Rev Cancer,* 2010, vol. 10 (2), 87-101
- **SPECHLER, S.J. et al.** American Gastroenterological Association technical review on the management of Barrett's esophagus. *Gastroenterology,* 2011, vol. 140 (3), e18-52
- **WANG, K.K. ; R.E. SAMPLINER.** Updated guidelines 2008 for the diagnosis, surveillance and therapy of Barrett's esophagus. *Am J Gastroenterol,* 2008, vol. 103 (3), 788-97
- **HIROTA, W.K. et al.** ASGE guideline: the role of endoscopy in the surveillance of premalignant conditions of the upper GI tract. *Gastrointest Endosc,* 2006, vol. 63 (4), 570-80
- **ARMSTRONG, D. et al.** Canadian Consensus Conference on the management of gastroesophageal reflux disease in adults - update 2004. *Can J Gastroenterol,* 2005, vol. 19 (1), 15-35
- **WATSON A, H.R.S.N.** *British Society of Gastroenterology, Guidelines for the diagnosis and management of Barrett's columnar-lined oesophagus,* 2005, http://www.bsg.org.uk
- **SHAHEEN, N.J. et al.** Radiofrequency ablation in Barrett's esophagus with dysplasia. *N Engl J Med,* 2009, vol. 360 (22), 2277-88
- **WANI, S. et al.** Esophageal adenocarcinoma in Barrett's esophagus after endoscopic ablative therapy: a meta-analysis and systematic review. *Am J Gastroenterol,* 2009, vol. 104 (2), 502-13
- **CURVERS, W.L. et al.** Endoscopic tri-modal imaging for detection of early neoplasia in Barrett's oesophagus: a multi-centre feasibility study using high-resolution endoscopy, autofluorescence imaging and narrow band imaging incorporated in one endoscopy system. *Gut,* 2008, vol. 57 (2), 167-72
- **KERKHOF, M. et al.** Grading of dysplasia in Barrett's oesophagus: substantial interobserver variation between general and gastrointestinal pathologists. *Histopathology,* 2007, vol. 50 (7), 920-7
- **MONTGOMERY, E. et al.** Reproducibility of the diagnosis of dysplasia in Barrett esophagus: a reaffirmation. *Hum Pathol,* 2001, vol. 32 (4), 368-78
- **WANI, S. et al.** Greater interobserver agreement by endoscopic mucosal resection than biopsy samples in Barrett's dysplasia. *Clin Gastroenterol Hepatol,* 2010, vol. 8 (9), 783-8
- **KAHRILAS, P.J.** The problems with surveillance of Barrett's esophagus. *N Engl J Med,* 2011, vol. 365 (15), 1437-8
- **SHAHEEN, N.J. et al.** Is there publication bias in the reporting of cancer risk in Barrett's esophagus?. *Gastroenterology,* 2000, vol. 119 (2), 333-8
- **BHAT, S. et al.** Risk of malignant progression in Barrett's esophagus patients: results from a large population-based study. *J Natl Cancer Inst,* 2011, vol. 103 (13), 1049-57
- **HVID-JENSEN, F. et al.** Incidence of adenocarcinoma among patients with Barrett's esophagus. *N Engl J Med,* 2011, vol. 365 (15), 1375-83
- **CURVERS, W.L. ; R. KIESSLICH ; J.J. BERGMAN.** Novel imaging modalities in the detection of oesophageal neoplasia. *Best Pract Res Clin Gastroenterol,* 2008, vol. 22 (4), 687-720
- **WOLFSEN, H.C. et al.** Prospective, controlled tandem endoscopy study of narrow band imaging for dysplasia detection in Barrett's Esophagus. *Gastroenterology,* 2008, vol. 135 (1), 24-31
- **SHARMA, P. et al.** Standard endoscopy with random biopsies versus narrow band imaging targeted biopsies in Barrett's oesophagus: a prospective, international, randomised controlled trial. *Gut,* 2012
- **KARA, M. et al.** Autofluorescence-based detection of early neoplasia in patients with Barrett's esophagus. *Dig Dis,* 2004, vol. 22 (2), 134-41
- **KARA, M.A. et al.** Endoscopic video autofluorescence imaging may improve the detection of early neoplasia in patients with Barrett's esophagus. *Gastrointest Endosc,* 2005, vol. 61 (6), 679-85
- **CURVERS, W.L. et al.** Endoscopic tri-modal imaging is more effective than standard endoscopy in identifying early-stage neoplasia in Barrett's esophagus. *Gastroenterology,* 2010, vol. 139 (4), 1106-14
- **CURVERS, W.L. et al.** Endoscopic trimodal imaging versus standard video endoscopy for detection of early Barrett's neoplasia: a multicenter, randomized, crossover study in general practice. *Gastrointest Endosc,* 2011, vol. 73 (2), 195-203
- **KAYE, P.V. et al.** Barrett's dysplasia and the Vienna classification: reproducibility, prediction of progression and impact of consensus reporting and p53 immunohistochemistry. *Histopathology,* 2009, vol. 54 (6), 699-712
- **SKACEL, M. et al.** p53 expression in low grade dysplasia in Barrett's esophagus: correlation with interobserver agreement and disease progression. *Am J Gastroenterol,* 2002, vol. 97 (10), 2508-13
- **MURRAY, L et al.** TP53 and progression fro m Barrett's metaplasia to oesophageal adenocarcinoma in a UKpopulation cohort. *Gut,* 2006, vol. 55 (10), 1390-7
- **SIKKEMA, M. et al.** Aneuploidy and overexpression of Ki67 and p53 as markers for neoplastic progression in Barrett's esophagus: a case-control study. *Am J Gastroenterol,* 2009, vol. 104 (11), 2673-80
- **SCHULMANN, K et al.** Inactivation of p16, RUNX3, and HPP1 occurs early in Barrett's-associated neoplastic progression and predicts progression risk. *Oncogene,* 2005, vol. 24 (25), 4138-48

- **GALIPEAU, P.C. et al.** NSAIDs mo dulate CDKN2A, TP53, and DNA c ontent risk for progression to e so p ha geal adenoc arcinoma. *PLo SMed,* 2007, vol. 4 (2), e67
- **LAO-SIRIEIX, P. ; L LOVAT ; RC. FITZGERALD.** Cyclin A immunocytology as a risk stra tific a tio n tool for Barrett's esophagus surveillance. *Clin Cancer Res,* 2007, vol. 13 (2), 659-65
- **GALIPEAU, P.C. et al.** Clonal expansion and loss of heterozygosity at chromosomes 9p and 17p in premalignant esophageal (Barrett's) tissue. *J Natl Cancer Inst,* 1999, vol. 91 (24), 2087-95
- **LEEDHAM, S.J. et al.** Individual crypt genetic heterogeneity and the origin of metaplastic glandular epithelium in human Barrett's oesophagus. *Gut,* 2008, vol. 57 (8), 1041-8
- **FUKASAWA, K et al.** Abnormalcentrosome amplification in the absence of p53. *Science,* 1996, vol. 271 (5256), 1744-7
- **THOMPSON, S.L ; D.A. COMPTON.** Proliferation of aneuploid human cells is limited by a p53-dependentmechanism. *J Cell Biol,* 2010, vol. 188 (3), 369-81